# EUROPEAN PATENT SPECIFICATION

(11) **EP 1 073 770 B1**
(45) Date of publication and mention of the grant of the patent: **17.11.2004**
(21) Application number: 99924858.6
(22) Date of filing: 30.04.1999
(51) Int. Cl.: C12Q 1/68, C12N 15/10

(54) **NOVEL METHOD FOR THE IDENTIFICATION OF CLONES CONFERRING A DESIRED BIOLOGICAL PROPERTY FROM AN EXPRESSION LIBRARY**
NEUARTIGES VERFAHREN ZUR IDENTIFIZIERUNG VON KLONEN MIT EINER GEWÜNSCHTEN BIOLOGISCHEN EIGENSCHAFT, AUSGEHEND VON EINER EXPRESSIONSGENBANK
NOUVEAU PROCEDE PERMETTANT L'IDENTIFICATION DE CLONES CONFERANT UNE PROPRIETE BIOLOGIQUE DESIREE DANS UNE BANQUE D'EXPRESSION

(30) Priority: 30.04.1998 US 70590
(43) Date of publication of application: 07.02.2001
(73) Proprietor: MAX-PLANCK-GESELLSCHAFT ZUR FÖRDERUNG DER WISSENSCHAFTEN E.V., 14195 Berlin (DE)
(72) Inventor: CAHILL, Dolores, D-14195 Berlin (DE); BÜSSOW, Konrad, D-12167 Berlin (DE); WALTER, Gerald, 06300 Nice (FR); LEHRACH, Hans, D-14195 Berlin (DE)
(74) Representative: Dreiss, Fuhlendorf, Steimle & Becker
(86) International application number: PCT/EP1999/002963
(87) International publication number: WO 1999/057311

(56) References cited:
- WO-A-97/32017
- WO-A-99/57312
- LEHRACH ET AL: "HIGH THROUGHPUT TOOLS FOR GENE IDENTIFICATION AND FUNCTIONAL GENE ANALYSIS" NUCLEIC ACIDS SYMPOSIUM SERIES,GB,IRL PRESS, OXFORD, vol. 38, no. 38, page 7-10-10 XP002114354 ISSN: 0261-3166
- MAIER ET AL: "AUTOMATED ARRAY TECHNOLOGIES FOR GENE EXPRESSION PROFILING" DRUG DISCOVERY TODAY,GB,ELSEVIER SCIENCE LTD, vol. 2, no. 8, page 315-324-324 XP002103832 ISSN: 1359-6446 cited in the application
- DAVIES C A AND BENZER S: "Generation of cDNA expression libraries enriched for in-frame sequences" PROCEEDINGS OF THE NATIONAL ACADEMY OF SCIENCES USA, vol. 94, 1997, pages 2128-2132, XP002114083
- LENNON G & LEHRACH H: "Hybridization analyses of arrayed cDNA libraries" TRENDS IN GENETICS, XP002095118 cited in the application
- LEHRACH H ET AL.: "Robotics, computing, and biology" INTERDISCIPLINARY SCIENCE REVIEWS, vol. 22, no. 1, 1997, pages 37-44, XP000863340 cited in the application
- LEFFERS H ET AL.: "cDNA expression and human two-dimensional gel protein databases: Towards integrating DNA and protein information" ELECTROPHORESIS, vol. 17, 1996, pages 1713-1719, XP000857621 cited in the application
- BÜSSOW ET AL: "A METHOD FOR GLOBAL PROTEIN EXPRESSION AND ANTIBODY SCREENING ON HIGH-DENSITY FILTERS OF AN ARRAYED cDNA LIBRARY" NUCLEIC ACIDS RESEARCH,GB,OXFORD UNIVERSITY PRESS, SURREY, vol. 26, no. 21, page 5007-5008-5008 XP002114084 ISSN: 0305-1048 cited in the application
- LUEKING A ET AL.: "Protein microarrays for gene expression and antibody screening" ANALYTICAL BIOCHEMISTRY, vol. 270, 1999, pages 103-111, XP002127382
- CLARK M D ET AL.: "Construction and analysis of arrayed cDNA libraries" METHODS IN ENZYMOLOGY, vol. 303, 1999, pages 205-233, XP000857457

## Description

Proteins are genomic sequence information translated into functional units, enabling biological processes. Initial attempts at sequencing the large and complex human genome were intentionally focused on expressed regions, as represented by cDNA repertoires (Adams et al., Nature 377 (1995), 3S-174S). Meanwhile, expressed sequence tags (ESTs) for most human genes have been deposited in the nucleotide databases (Wolfsberg et al., Nucl. Acids Res. 25 (1997), 1626-1632). However, only a minority of these sequences have yet been assigned a function (Strachan et al., Nature Genet. 16 (1997), 126-132). The most straightforward solution to this structure-function discrepancy seems to be the direct correlation between the functional status of a tissue and the expression of certain sets of genes. Technology is now available to approach this goal on different levels of gene expression. On the transcriptional level, gene expression patterns have been analyzed by hybridization of complex probes (DeRisi et al., Science 278 (1997), 680-686; Schena et al., Science 270 (1995), 467-470; Bernard et al., Nucl. Acids Res. 24 (1997), 1435-1442; Mallo et al., Int. J. Cancer 74 (1997), 35-44) or sets of short oligonucleotides (Velculescu et al., Science 270 (1995), 484-487) to cDNA arrays, the SAGE sequencing approach (Wodicka et al., Nature Biotechnol. 15 (1997), 1359-1367) or hybridization to oligonucleotide arrays (Maier et al., Drug Discovery Today 2 (1997), 315-324).
On the translational level, protein extracts have been mapped at high resolution on two-dimensional gels (Klose et al., Electrophoresis 16 (1995), 1034-1059). Mass spectrometry analysis of protein spots was then used to obtain sequence information (Clauser et al., Proc. Natl. Acad. Sci. USA 92 (1995), 5072-5076). Clonal cDNA expression in mammalian cells and matching of the protein products to two-dimensional electrophoresis patterns of cellular proteins was described by Leffers et al. (Leffers et al., Electrophoresis 17 (1996), 1713-1719). Pooled clones from an ordered cDNA library were expressed by *in vitro* transcription/translation and analyzed by two-dimensional electrophoresis (Lefkovits et al., Appl. Theor. Electrophor. 5 (1995), 35-42; Behar et al., Appl. Theor. Electrophor. 5 (1995), 99-105; Lefkovits et al., Appl. Theor. Electrophor. 5 (1995), 43-47). Lehrach et al. (Nucl. Acids Res. 38, (1998), 8) described arrays of clones of cDNA libraries on high density filter grids. Nowhere does Lehrach et al., however, teach or suggest to use libraries of fusion proteins as required by the present invention.
The construction and screening of fusion protein libraries has been described in lkeda et al (Gene 181, (1996) 167-171). lkeda et al. does not, however, describe or suggest clones or proteins arrayed in grid form.
Until now, no technique has been available to go directly from DNA sequence information on individual clones to protein products and back again at a whole genome level. Such a method would in particular be important for the large scale analysis of biological material.

Rather, the prior art methods devised for the large scale analysis of such material are quite laborious as well as time consuming and, in addition, as a rule provide an inappropriately large number of false positive clones. Accordingly, the technical problem underlying the present invention was to provide a method that overcomes the above mentioned problems and, in particular, significantly reduces the number of false positive clones in library screens especially on the level of mammalian genomes. The solution to said technical problem is achieved by providing the embodiments characterized in the claims.

### SUMMARY OF THE INVENTION

The present invention relates to a novel method for the identification and/or characterization of clones conferring a desired biological property from an expression library. The method of the invention comprises the step of analyzing for the expression of at least one (poly)peptide, such as a tag expressed as a fusion protein, together with a recombinant insert of a clone of said expression library, wherein the clones of said expression library are arranged in arrayed form. Said (poly)peptide may be fused N-terminally or C-terminally to said insert. The method of the invention further comprises the steps of contacting a ligand specifically interacting with a (poly)peptide expressed by the insert of a clone conferring said desired biological property with a first replica of said library of clones in arrayed form and analyzing said library of clones for the occurrence of an interaction, and/or carrying out a hybridization or an oligonucleotide fingerprint with a nucleic acid probe specific for the insert of a clone conferring said desired biological property with a second replica of said library of clones arranged in arrayed form and analyzing said library of clones for the occurrence of a specific hybridization. Finally, the method of the invention requires the identification of clones wherein an expression of the at least one (poly)peptide in step (a) and/or an interaction in step (b) and/or a hybridization or an oligonucleotide fingerprint in step (c) can be detected. The present invention also relates to a kit useful for carrying out the method of the invention.

### DETAILED DESCRIPTION OF THE INVENTION

The present invention relates to a method for the identification and/or characterization of clones of an expression library, said clones conferring a desired biological property comprising the following steps
(a) analyzing for the expression of at least one (poly)peptide expressed as a fusion protein with an expression product of a recombinant insert of a clone of said expression library, the clones of said expression library being arranged in arrayed form; and
(b) contacting a ligand specifically interacting with a (poly)peptide expressed by the insert of a clone conferring said desired biological property with said library or a first replica of said library of clones in arrayed form and analyzing said library of clones for the occurrence of an interaction; and/or
(c) carrying out a hybridization or an oligonucleotide fingerprint with a nucleic acid probe specific for the insert of a clone conferring said desired biological property with said library or said first replica or a second replica of said library of clones arranged in arrayed form and analyzing said library of clones for the occurrence of a hybridization; and
(d) identifying and/or characterizing clones wherein the expression of the at least one (poly)peptide in step (a) and/or an interaction in step (b) and/or a hybridization or an oligonucleotide fingerprint in step (c) can be detected.

The term "recombinant insert" as used in accordance with the present invention denotes a nucleic acid fragment which is present in the expression vector used for the preparation of said expression library such that it yields an open reading frame together with the nucleic acid fragment encoding said at least one (poly)peptide, the expression of said open reading frame resulting in said fusion protein.
The term "clone of an expression library" as used in connection with the present invention denotes any propagable, essentially clonal biological material that contains recombinant genetic material and is part of an expression library. Typically, this term will refer to bacterial transformants but may also relate to other transformants or to recombinant viral material or bacteriophage. The term "expression library" is well understood in the art; see, for example, Sambrook et al., "Molecular Cloning, A Laboratory Handbook", 2^{nd} edition (1989), CSH Press, Cold Spring Harbor, N.Y. Preferably, the expression library can be induced by an inductor. Inductors are known in the art and include, for example, IPTG. Various types of expression libraries are known in the art. All of these types are encompassed by the present invention. A preferable type of library is a library resulting from exon trapping, i.e. an exon trapped library, or a library made in a shuttle vector, for example, a vector which can be used in prokaryotic and eukaryotic systems, or in multiple prokaryotic and/or in multiple eukaryotic systems. Further, it is well known that expression libraries can be constructed from a large variety of sources. Again, the present invention envisages the use of all said sources in the above mentioned method. Such sources may be, for example, mammalian or other eukaryotic cells, tissue, bacteria, other microorganisms, plant, yeast, blood, or cell lines.
The term "desired biological property" is intended to encompass functional as well as non-functional biological properties such as structural properties. Functional properties may, for example, be binding properties as conferred by antibodies or fragments or derivatives thereof. In another alternative, said functional properties may relate to the turnover of target-molecules, such as provided by enzymatic activities. On the other hand, non-functional properties may relate to the primary structure of a nucleic acid that can be detected, for example, by nucleic acid hybridization.
The term "(poly)peptide" refers both to peptides and to polypeptides, naturally occurring or recombinantly, chemically or by other means produced or modified, which may assume the three-dimensional structure of proteins and may be post-translationally processed, optionally in essentially the same way as native proteins.
The term "fusion protein" denotes any polypeptide consisting or comprising of at least two (poly)peptides not naturally forming such a polypeptide. On the DNA level, the two or more coding sequences are fused in frame.
The term "arrayed form" as used herein refers to any regular or non-regular form that can be replicated. Preferred are regular forms, in particular high density grids as described, for example, in Lehrach et al., Interdisciplinary Science Reviews 22 (1997), 37-44.
The term "ligand" as used herein comprises any type of molecule that is, by way of its three-dimensional structure, capable of specifically interacting with a desired (poly)peptide. Depending on its three-dimensional structure, said ligand may also interact non-specifically with (poly)peptides expressed by the recombinant inserts. A typical example of a ligand is an antibody or another receptor such as a hormone receptor. Regarding antibodies, a typical example of a non-specific interaction is a cross-reaction.
The term "hybridization" with a nucleic acid probe refers to specific or non-specific hybridization. Whether a hybridization is specific or non-specific depends on the stringency conditions, as is well known in the art. The term "specific hybridization" relates to stringent conditions. Said hybridization conditions may be established according to conventional protocols described, for example, in Sambrook, "Molecular Cloning, A Laboratory Handbook", 2^{nd} edition (1989), CSH Press, Cold Spring Harbor, N.Y.; Ausubel, "Current Protocols in Molecular Biology", Green Publishing Associates,and Wiley Interscience, N.Y. (1989); or Higgins and Hames (eds) "Nucleic acid hybridization, a practical approach" IRL Press Oxford, Washington DC (1985). An example for specific hybridization conditions is hybridization in 4 x SSC and 0.1 % SDS at 65°C with subsequent washing in 0.1 x SSC, 0.1% SDS at 65°C. Alternatively, stringent hybridization conditions are, for example, 50% formamide, 4 x SSC at 42°C. Non-specific conditions refer, for example, to hybridization in 4 x SSC, 1% SDS at 50°C and washing at the same conditions.
In accordance with the present invention step (b) and/or (c) can be performed with said library and/or a first replica and/or a second and/or a further replica of said library. If said library or said first or second or further replica is used in two different steps, any material added during the step (a) and/or (b) which may interfere with the subsequent step(s) may, optionally, be removed prior to the performance of the subsequent step, preferably according to conventional protocols.
The term "identifying clones" comprises all types of identification steps suitable to identifying the clone of interest. For example, clones may be identified by visual means, for example, if the (poly)peptide expressed as a fusion protein with the recombinant insert is Green Fluorescent Protein and the ligand or the probe are labeled with a visually detectable label, e.g., alkaline phosphatase, horseradish peroxidase, or FITC. Furthermore, positive clones may be identified by the blue/white selection which is well known in the art. Alternatively, if the nucleic acid probe is marked with a radioactive label, exposure to an X-ray film may help identifying the desired clone. The clones may also be identified using mass spectrometry.
The term "oligonucleotide fingerprinting" describes generating a sequence dependent, reproducible, statistically significant pattern or fingerprint of the sequence obtained by analyzing the hybridization pattern (hybridization/no hybridization) obtained on hybridizing a number of oligonucleotides onto the nucleic acid, preferably DNA.

The method of the invention displays significant advantages over prior art methods and is particularly suitable for the efficient analysis of mammalian and/or plant and/or other eukaryotic genomes but can, of course, also be applied to the analysis of other expression libraries, e.g., genomic DNA expression libraries from prokaryotic or other microorganisms. The new method significantly reduces the background of false-positive clones in expression library screening. Especially when large numbers of clones within one or more libraries are screened, the time consuming work of identifying clones that eventually turn out to not have the desired biological properties can be avoided. This, of course, will also lead to a significant reduction of the cost factor in genomic and/or proteomic analysis. A further particular advantage of the present invention is that the investigator has the choice to select between a nucleic acid probe and a ligand for screening his library for the desired clones. The combination of steps (a), (b), and (c) will further enhance the reliability of the method of the invention for identifying the actually desired clones. Surprisingly, it could be shown in accordance with the invention, that, upon the original spotting of transformants in an array, and the subsequent growth of colonies, said detectable (poly)peptide can still be detected without disturbance of the array structure. This holds also true if the colonies have been cultivated for about 18 hours.

As regards the (poly)peptide expressed as a fusion protein with a recombinant insert of a clone of said expression library, it is to be noted that the present invention envisages the use of one or more of said (poly)peptides incorporated into said fusion protein. As is apparent from the appended examples, fusion of the (poly)peptide to the N-terminus allows for the detection of inserts that are expressed in frame since, as a rule, inserts which are not in frame with the N-terminal (poly)peptide will be rapidly degraded within the cytoplasm. On the other hand, the fusion of said (poly)peptide to the C-terminus and detection of said (poly)peptide allows for the selection of full-length inserts. Also, the present invention envisages the combination of one or more (poly)peptides fused to the N-terminal and C-terminal end of the insert.

It is to be noted that prior to carrying out steps (a) to (d) the clones should present the biological material to be tested for in an accessible form. If the clones are, for example, bacterial transformants, said transformants would preferably have to be lysed. Such lysis methods are well known in the art.
The application of computer-related technology with the method of the invention allows for the fact that screening needs to be done only once for a library. This is because data produced for individual clones by a later analysis, e.g. sequencing, can be related back to this screening. Accordingly, a rapid transition from an expression library such as a cDNA library to a protein library has become possible. This creates a direct link between a gene catalogue and a functional protein/(poly)peptide catalogue. In addition to the above, a repeated screening of or a prolonged screening reaction may further enhance the chance of excluding false-positive clones.

In accordance with the present invention the method may also be used to characterize already known nucleic acid molecules.

In a preferred embodiment of the method of the invention, said (poly)peptide expressed as a part of a fusion protein with said expression product of said recombinant insert is an antibody or a fragment or derivative thereof wherein said fragment is a F(ab')₂, Fab, Fv or scFv fragment or an antibody modified such as by deletion of certain parts thereof but essentially maintaining its capacity to function as a ligand, a tag, an enzyme, or a phage protein or a fusion protein.

Methods for detecting any embodiment of the above specified (poly)peptide are well known in the art or can be devised by the person skilled in the art without further ado. For example, antibodies can be detected by anti-antibodies which are detectably labeled. As regards the antibody fragments or derivatives thereof, these may include F(ab')₂, Fab, Fv or scFv fragments; see, for example, Harlow and Lane, "Antibodies; A Laboratory Manual", CHS Press (1988), Cold Spring Harbor, N.Y. Further, tags may be detected according to conventional methods. The same holds true for enzymes which may be detected, for example, by reacting the same with a specific substrate and detecting, for example, a color reaction, or by using a detectably labeled antibody specific for said enzyme. Antibodies may also be used to detect phage or fragments thereof. Labels for antibodies are also well known in the art and include alkaline phosphatase (ATTPPHOS), CSPD, horseradish peroxidase, FITC, and radioactivity. Also, mass spectrometry can be used for detecting any embodiment of the above specified (poly)peptide.

In a further preferred embodiment of the method of the invention, said analysis for the expression of a (poly)peptide in step (a) is effected by contacting a ligand different from the ligand of step (b) that specifically interacts with said (poly)peptide and analyzing said library of clones for a specific interaction to occur. The ligand used in step (a) may be the same class of ligand that is used in step (b). However, the actual molecular structure of the ligand should be different in both steps in order to be able to differentiate between the two ligands.

In an additional preferred embodiment of the method of the invention, said analysis for the expression of a (poly)peptide in step (a) is effected by visual means.
Advantageously, the expression of said (poly)peptide can be detected by visual means such as by fluorescence, bioluminescence or phosphorescence. The corresponding signals may be stored by photographic means which may be attached to a computer unit. The corresponding signals may be imaged using a high resolution CCD detection system, saved and stored on computer as image files and analyzed using custom written software to score positive clones.

It is most preferred that said visual means employ mass spectrometry. For example, here mass spectrometry analysis of the arrayed proteins allows the use of the protein arrays as a bridge to link DNA, mRNA, and/or complex hybridization results to 2-D-PAGE results. This is done by generating mass spectra of the arrayed proteins (e.g. on a chip, a mass spectrometry target or a matrix), and comparing these mass spectra with mass spectra generated from spots on 2-D gels. Using this approach, the mRNA repertoire of a cell (via the cDNA library) may be studied as the first level of gene expression, which most directly reflects gene activity, and may be related to proteome analysis which is the analysis of the protein complement of a cell, tissue, plant, microorganism and/or organism.

Currently, the isolated proteins from 1-D and 2-D gels are identified in sequence databases using mass spectrometry. Clearly, however, this is limited to the few known proteins. Advantageously, this limitation is overcome by the concept of the present invention, namely that each protein, expressed by the clones of the expression libraries, is specified by a minimal set of structural information, which is designated "minimal protein identifier" (MPI). The content of MPls, peptide maps combined with additional structural data, may be optimized in two ways, for unambiguous protein identification and for high throughput determination by mass spectrometry.

Once recorded, MPIs facilitate tracing gene products in biological samples, simply by comparing the measured data. In this way, protein recognition is independent of whether the protein is "known" (i.e. present in the current databases) or "unknown" (i.e. not present in the current databases). These spectra can be used to identify spectra subsequently generated from the analysis of protein from other sources, e.g. such as from separated proteins from 1-D and 2-D electrophoresis gels.

This provides a bridge that connects the proteins characterized by 2-D electrophoresis, with their corresponding mRNAs and genes (cDNAs). All MPls collected from 2-D gels are compared by computer-based methods (*in silico*) with the MPls obtained from the recombinant protein library, and vice-versa. Thereby, thousands of biologically active gene products can be linked to their genes. This linkage is independent of any sequence information and, therefore, also attractive for functional proteome analysis of other organisms.

Another advantage of the strategy of the present invention, compared to current strategies, is that protein identification becomes more reliable because mass spectrometric data are compared with mass spectrometric data, and not with data predicted from DNA or protein sequences. Major shortcomings of the latter approach are that substrate dependent protease performance, peptide solubility, and final signal suppression in the mass spectrometric analysis are not considered.

Furthermore, the protein arrays of the present invention allow exploring mass spectrometric data of thousands of different proteins taken from 2-D gels by using their recombinant homologues labeled with stable-isotopes. In addition, it provides an immortal source for generating cDNA microarrays to be used to profile mRNA levels by complex hybridization.

In another preferred embodiment of the method of the invention, said biological property is specificity for a cell, a tissue, or the developmental stage of a cell or a tissue, a microorganism, preferably a bacterium, a plant or an organism.
In this preferred embodiment of the invention, specific comparisons can be made that provide the investigator with information, for example, with respect to the developmental status of a cell, a tissue, or an organism, or the specificity of a cell or a tissue, for example, with respect to its origin. This can be done by comparing two tissues from different origins for the presence of certain marker proteins. For example, with respect to the developmental status of an organism expression profiles of a 6-day old mouse embryo arrayed cDNA expression library and a 9-day old mouse embryo arrayed cDNA expression library may be compared to identify and characterize differentially expressed genes, thereby elucidating proteins expressed at different stages of development.

In a further preferred embodiment of the method of the invention, said cell or tissue is a normal cell or tissue, a diseased cell or tissue, or a pretreated cell or tissue.

The term "pretreated" as used in combination with cell or tissue is intended to mean that said cell or tissue has been exposed to a drug, an activator or a ligand etc. Said pretreatment will have, as a rule, affected the cellular pathways and optionally resulted in at least one phenotypic change as compared to a not pretreated cell. It is envisaged that said at least one phenotypic change is detected using the method of the invention. Also, it is expected that diseased tissue or cells display phenotypic differences as compared to healthy tissues or cells that can be detected with the method of the invention.

In another preferred embodiment of the method of the invention, said clones are bacterial transformants, recombinant phage, transformed mammalian, insect, fungal, yeast or plant cells.

Bacterial transformants are preferably transformed E. coli cells; recombinant phage is preferably derived from M13 or fd phage; transformed or transfected mammalian cells may be Hela or COS cells. As regards insect cells, Spodoptera frugiperda or Drosophila melanogaster cells are preferred. Preferred fungal cells comprise Aspergillus cells whereas said yeast cells are preferably derived from Pichia pastoris or Saccharomyces cerevisiae. It is to be noted that the terms "transformed" and "transfected" are used interchangeably in accordance with this invention.

In the case that said bacterial transformants are transformed E. coli cells, it is most preferred that E. coli SCS1 cells as described in the Examples, infra, are used. In another most preferred embodiment, the E. coli cells are transformed with a library cloned into a vector allowing an inducible expression, preferably also expressing a tag as part of said fusion protein, preferably vector pQE-30NST as described in the Examples, infra. However, the person skilled in the art is well aware of the structural and/or functional features of the E. coli cells and/or vectors as described in the Examples such that any E. coli cells and/or vectors displaying essentially the same structural and/or functional features are encompassed by the present invention.

Another preferred embodiment of the invention relates to a method, wherein said arrayed form has substantially the same format in steps (a) to (c).
This embodiment of the invention is particularly useful since it allows for the production of replicas from one master plate and the comparison of results on a 1:1 scale. On the other hand and less preferred, the arrayed form may have a different format such as a different scale in at least two of steps (a) to (c) as long as the unambiguous relation of clones on the various replicas is still possible.

In a further preferred embodiment of the method of the invention, said arrayed form is a grid form.

The grid should, in accordance with the discussion herein above, preferably allow for the high density array of clones of the expression library. It should further preferably have the format of grids that have been described in Lehrach, loc. cit.

In a most preferred embodiment of the method of the invention, said grid has the dimensions of a microtiter plate, a silica wafer, a chip, a mass spectrometry target or a matrix.

Using these dimensions, conventional laboratory material can be employed in the process of the invention. Additionally, these dimensions allow for the convenient analysis of a large number of clones on small scale equipment.

in another preferred embodiment of the method of the invention, said clones are affixed to a solid support.

The solid support may be flexible or inflexible. This embodiment in particular allows for the convenient storage and transport of the arrayed clones of the expression library. A particularly preferred embodiment refers to freeze dried clones that are affixed to said solid support.

A further preferred embodiment of the method of the invention relates to a method wherein said solid support is a filter, a membrane, a magnetic bead, a silica wafer, glass, metal, a chip, a mass spectrometry target or a matrix.

As regards filters or membranes, it is particularly preferred that they are produced from PVDF or Nylon. As regards filters or membranes, it is particularly preferred that DNA or DNA-containing clones are spotted/gridded/grown on Nylon membrane filters (for example, Hybond N+, Amersham) as this has a high DNA binding capacity and that proteins or protein-expressing clones are spotted/gridded/grown on polyvinylidene difluoride (PVDF) membrane filters (for example, Hybond PVDF, Amersham) as this has a high protein binding capacity.

In a further preferred embodiment of the method of the invention, at least one of said ligands is a (poly)peptide, a phage, blood, serum, a toxin, an inhibitor, a drug or a drug candidate, a non-proteinaceous or partially proteinaceous receptor, a catalytic polymer, an enzyme, a nucleic acid, a PNA, a virus a cell , an inorganic compound, a conjugate, a dye, a tissue or a conjugate of said ligand.

Accordingly, the ligand can be of a variety of natures. Importantly, the various types of ligands can be detected directly or indirectly and, thus, allow the identification of the desired clones.

In another preferred embodiment of the method of the invention, said (poly)peptide is an antibody or a F(ab')₂, Fab, Fv or scFv fragment, a hormone or an enzyme or an antibody, hormone or enzyme modified such as by deletion of certain parts thereof but essentially maintain their capacity to function as a ligand.

The above preferred (poly)peptides are especially versatile, easy to handle and can be provided in large different numbers.

In a further preferred embodiment of the method of the invention, said interaction in step (b) is a specific interaction.

An example of this situation is the case where an antibody binds specifically to one epitope or (poly)peptide sequence, for example, the anti-histidine antibody binds specifically the 6x-histidine tag, 5x-histidine tag, RGS-6x-histidine tag or to an epitope which is only found on one protein.

In an additional preferred embodiment of the method of the invention, said interaction in step (b) is an unspecific interaction.

An example of this situation is the case where an antibody binds non-specifically to epitopes which are not coded from identical DNA sequences but share similar three-dimensional structure, charge, etc. and can be present on different proteins. As could be demonstrated in accordance with the present invention, an application of this invention can be to determine the specificity or cross-reactivity of ligands such as antibodies. The detection of antibody cross-reactivities on protein microarrays is not surprising as antibodies are not usually tested against whole libraries of proteins. The method of the present invention for screening antibodies against arrays of potential antigens to detect common epitopes may be particularly important for reagents that are to be used for immunohistochemistry or physiological studies on whole cells or tissues, where they face batteries of different structures. Alternatively or additionally, antibodies with no known antigen specificity (e.g., lymphoma proteins) can be screened for binding to a highly diverse repertoire of protein molecules. As all of these proteins are expressed from isolated clones of arrayed cDNA libraries, the corresponding inserts can easily be sequenced to identify antigen-encoding genes. It is envisaged in accordance with the present invention to use the method for characterizing the binding and/or non-specificity of antibodies, serum, etc., for homology studies on protein families, and/or for defining binding domains and epitopes. Furthermore, the technique is not limited to antigen-antibody screening but may be applied to any ligand-receptor system.

In another preferred embodiment of the method of the invention, said hybridization in step (c) occurs under stringent conditions. It is alternatively preferred that said hybridization in step (c) occurs under non-stringent conditions.
With respect to the significance and applications of the stringent/non-stringent hybridizations, essentially the same applies as was set forth in connection with the discussion of the specific/unspecific interactions.

In a particularly preferred embodiment of the method of the invention, said tag is c-myc, His-tag, FLAG, alkaline phosphatase, V5 tag, T7 tag, or Strep-tag, a fusion protein, preferably GST, cellulose binding domain, green fluorescent protein, maltose binding protein or lacZ. In accordance with the invention, two or more tags may be comprised by the fusion protein.

The expression library employed in the method of the invention may be constructed from a variety of sources. For example, it may be a genomic library or an antibody library. Preferably said library of clones comprises a cDNA library.

The arrayed form is preferably generated using an automated device.

In a particular preferred embodiment of the method of the invention, said arrayed form of said library and/or said replicas is/are generated by a picking robot and/or spotting robot and/or gridding robot.

Another preferred embodiment of the present invention relates to a method further comprising sequencing the nucleic acid insert of said desired clone. Sequencing of said clone will, in many cases, provide the ultimately desired information obtainable with the method of the invention. Protocols for sequencing DNA or RNA are well known in the art and described, for example, in Sambrook, loc. cit.

In a final preferred embodiment of the method of the invention, the method comprises identifying the (poly)peptide encoded by the insert of the desired clone.
identification of said (poly)peptide expressed from the desired clone can be effected by a variety of methods. Such methods are known inter alia, as standard biochemical methods, such as affinity chromatography, SDS-PAGE, ELISA, RIA, etc. Once the (poly)peptide has been sufficiently characterized, a corresponding chemical component may be devised for pharmaceutical applications, e.g. by peptidomimetics.

It is to be understood that the introduced inserts and vectors express the gene product after introduction into said cell and preferably remain in this status during the lifetime of said cell. For example, cell lines which stably express the polynucleotide under the control of appropriate regulatory sequences may be engineered according to methods well known to those skilled in the art. Rather than using expression vectors which contain viral origins of replication, host cells can be transformed with the polynucleotide of the invention and a selectable marker, either on the same or separate plasmids. Following the introduction of foreign DNA, engineered cells may be allowed to grow for 1-2 days in an enriched media, and then are switched to a selective media. The selectable marker in the recombinant plasmid confers resistance to the selection and allows for the selection of cells having stably integrated the plasmid into their chromosomes and grow to form foci which in turn can be cloned and expanded into cell lines. Such engineered cell lines are also particularly useful in screening methods for the detection of compounds involved in, e.g., B-cell/T-cell interaction.

A number of selection systems may be used, including but not limited to the herpes simplex virus thymidine kinase (Wigler, Cell 11(1977), 223), hypoxanthine-guanine phosphoribosyltransferase (Szybalska, Proc. Natl. Acad. Sci. USA 48 (1962), 2026), and adenine phosphoribosyltransferase (Lowy, Cell 22 (1980), 817) in tk⁻, hgprt- or aprt⁻ cells, respectively. Also, antimetabolite resistance can be used as the basis of selection for dhfr, which confers resistance to methotrexate (Wigler, Proc. Natl. Acad. Sci. USA 77 (1980), 3567; O'Hare, Proc. Natl. Acad. Sci. USA 78 (1981), 1527), gpt, which confers resistance to mycophenolic acid (Mulligan, Proc. Natl. Acad. Sci. USA 78 (1981), 2072); neo, which confers resistance to the aminoglycoside G-418 (Colberre-Garapin, J. Mol. Biol. 150 (1981), 1); hygro, which confers resistance to hygromycin (Santerre, Gene 30 (1984), 147); or puromycin (pat, puromycin N-acetyl transferase). Additional selectable genes have been described, for example, trpB, which allows cells to utilize indole in place of tryptophan; hisD, which allows cells to utilize histinol in place of histidine (Hartman, Proc. Natl. Acad. Sci. USA 85 (1988), 8047); and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.).

The invention also relates to a kit comprising at least two replicas of expression libraries as referred to herein above affixed to a solid support wherein the clones of such expression libraries are arranged in grid form. The kit of the invention is particularly suitable for carrying out the method of the invention. The various types of possible and preferred solid supports have been defined herein above. Preferably, the kit of the present invention further comprises at least one ligand as defined hereinabove.

The components of the kit of the invention may be packaged in containers such as vials, optionally in buffers and/or solutions. If appropriate, one or more of said components may be packaged in one and the same container.

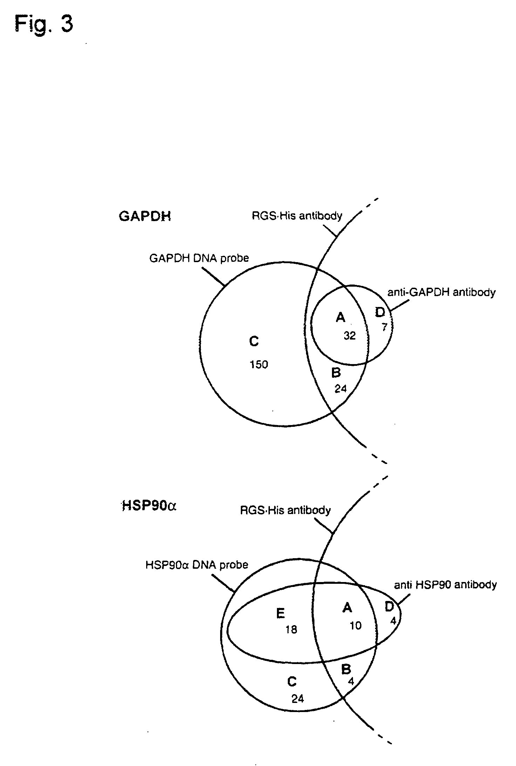

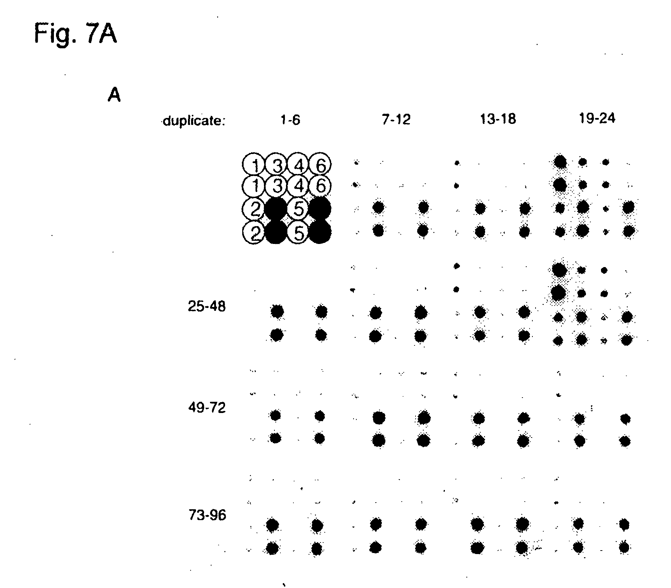

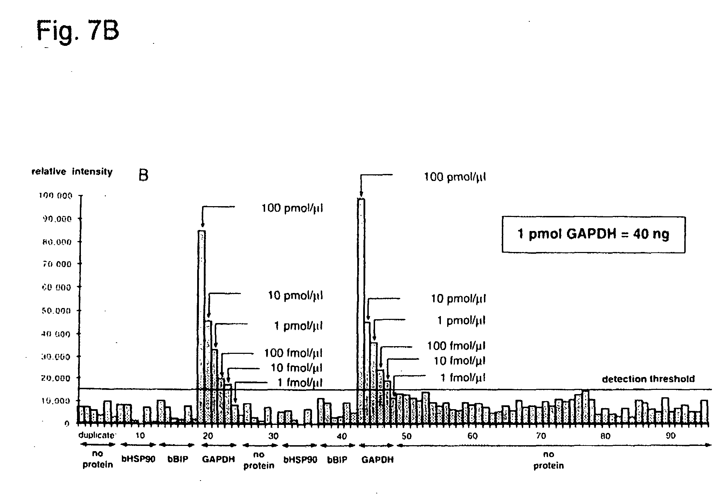

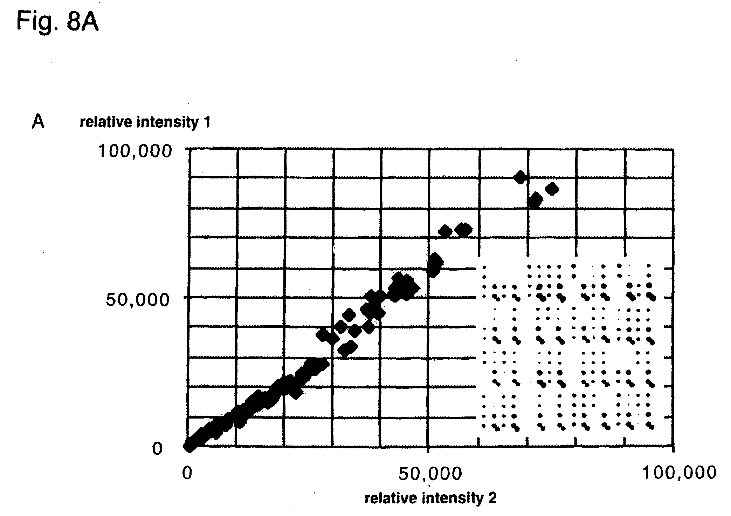

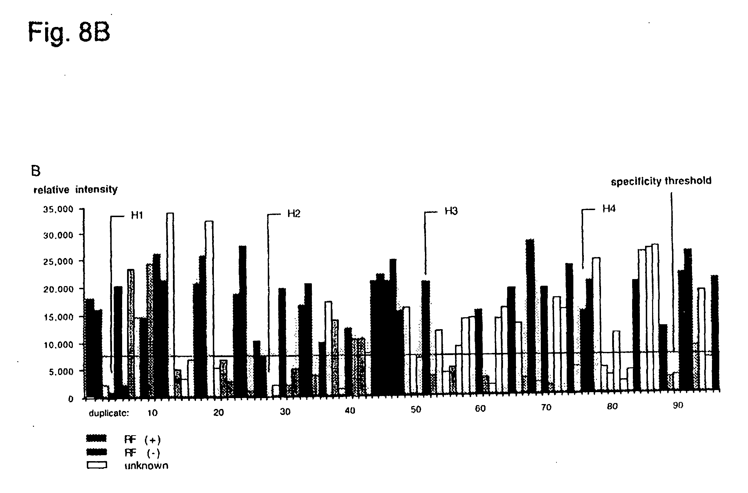

The figures show:
**Figure 1**
   RGS·His detection of protein expression clones with the RGS·His antibody on a high-density filter. A filter displaying 27,648 clones, arrayed in duplicate, was screened with the RGS·His antibody to detect clones expressing His6-tagged recombinant proteins.
**Figure 2**
   Identification of GAPDH expression clones. (a) Screening of a DNA filter representing 27,648 cDNA clones, arrayed in duplicate, with a GAPDH-specific DNA probe. (b) Screening of an identical protein filter representing the same clones as in (a) with an anti-GAPDH antibody. Corresponding sections of filters are shown.
**Figure 3**
   Venn diagrams showing the categories of clones identified by different probes and antibodies. Circles represent sets of clones identified by individual probes. Clones in intersections were detected by multiple probes.
**Figure 4**
   Sequence alignments of sequences of GAPDH (a) and HSP90a (b) clones. The open reading frames of GAPDH and HSP90a are shown as open boxes. Each line indicates the length of the sequence expected to be present in the respective clone, with thicker sections showing the fragment actually sequenced and aligned to the full-length mRNA sequence. The letters A-E refer to the categories in Fig. 3.
**Figure 5**
   Protein products of clones detected by RGS·His and/or specific antibodies against GAPDH (a) or HSP90a (b). Shading and numbers in the boxes across the top indicate signal intensities on high-density filters. Whole cellular proteins were stained with Coomassie blue. Clone categories are the same as in Fig. 3.
**Figure 6**
   Transfer stamp for protein solution transfer from 384-well microtitre plates to PVDF membranes. 16 individual, spring-loaded, stainless steel pins are mounted into a POM (Polyoxymethylene, Polyformaldehyde, Polyacetale) corpus. The pin-to-pin distance is 4.5 mm. The blunt end tip size was measured to 250 µm.
**Figure 7**
   Sensitivity of specific protein detection on microarrays. Equimolar concentrations (100 pmol/µl - 1 fmol/µl) of purified human GAPDH (duplicates 19-24 and 43-48), human bHSP90alpha (duplicates 7-12 and 31-36) and rat bBIP (duplicates 13-18 and 37-42) were spotted (5 x 5 nl) in two identical series of duplicates and detected using a monoclonal anti-GAPDH antibody. A: Spot array on PVDF filter membrane (1.9 x 1.9 cm holding 128 samples, 4 x 4 vertical duplicate spotting pattern, black duplicate guide spots, counting of duplicates as indicated); B: Relative intensities of means of duplicates in A (guide spots excluded), indicating numbering of duplicates (as in A), name and amounts of protein spotted and detection threshold.
**Figure 8**
   High-throughput expression of RGS-His₆-tagged fusion proteins from clones of the arrayed hEx1 library as detected on a microarray using the monoclonal antibody RGS-His (Qiagen). Crude, filtered lysates of 92 clones were spotted from a 96-well microtitre plate, including 4 wells with control proteins (H1, vector pQE-30NST without insert; H2, bHSP90alpha, clone N15170, vector pQE-BH6; H3, GAPDH, clone D215, vector pQE-30NST; H4, bBIP, vector pQE-BH6). A: Reproducibility of detection as diagonal of relative intensities of duplicates; Insert: Spot array on PVDF filter membrane (as in Fig. 7, lower guide spots doubled for orientation); B: Diagramme as in Fig. 7, indicating (+) or (-) Reading Frames of inserts if known (specificity threshold arbitrarily set to 7,500 relative intensity).
**Figure 9**
   Specificity testing of three monoclonal antibodies on identical microarrays of RGS-His₆-tagged fusion proteins expressed from clones of the arrayed hEx1 library as in Fig.8. A: monoclonal anti-GAPDH (H3, GAPDH, clone D215, vector pQE-30NST); B: monoclonal anti-HSP90alpha (H2, bHSP90alpha, clone N15170, vector pQE-BH6; H10, 60S ribosomal protein L18A; H3, GAPDH, clone D215, vector pQE-30NST); C: monoclonal anti-alpha tubulin (F9 and A4, RF(+) alpha tubulin clones; C7, RF(-) alpha tubulin clone; B1 and B12, unknown genes; H3, GAPDH, clone D215, vector pQE-30NST; G1, RF(-) beta tubulin clone; E5, RPL3 ribosomal protein L3; H10, RPL18A ribosomal protein L18A; E6 and D8, RPS2 ribosomal protein S2; F7, RPS3A ribosomal protein S3A; E3, RPS25 ribosomal protein S25); specificity threshold arbitrarily set to 25,000 relative intensity.

### Table legends:

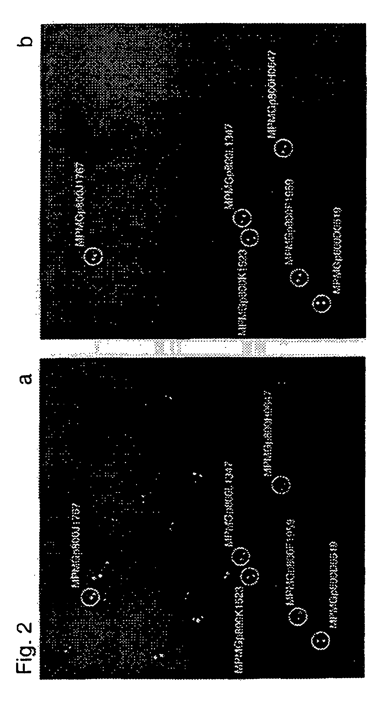

**Table 1**
   Evaluation of different screening options for the hEx1 cDNA expression library. Clone categories are as in Fig. 3. Numbers in brackets represent second screenings.
**Table 2**
   Evaluation of different screening options for the hEx1 cDNA expression library. Clone categories are as in Fig. 2. Numbers in brackets represent second screenings.

The following examples are intended to illustrate the invention.

### Example 1: Construction of an arrayed human cDNA expression library

A directionally cloned human fetal brain cDNA library (hEx1) was constructed in pQE 30NST, a vector for IPTG-inducible expression of His6-tagged fusion proteins. pQE30-NST was constructed from pQE-30 (Qiagen), a pBR322-based expression vector that carries a phage T5 promoter and two lac operators for IPTG-inducible recombinant protein expression as follows; in the first step, pQE-30N was generated by inserting a synthetic oligonucleotide carrying a BgIII and a Notl site into the unique Pstl site of pQE-30. In subsequent steps, an SP6 promoter oligonucleotide carrying an SP6 promoter was inserted between the BamHl and the Sall site of pQE-30N, followed by insertion of a second oligonucleotide carrying a T7 promoter between the Hindlll and the Notl site. The resulting vector, pQE-30NST, can be used for cloning of cDNAs with Sall and Notl overhangs. The insert can be transcribed in vitro in sense direction using SP6 RNA polymerase and in antisense direction using T7 RNA polymerase.
An average insert size of about 1.4 kb was obtained by PCR analysis of 14 clones.
E. coli SCS (Stratagene) carrying pSE111 was used as the host strain to construct this expression library. pSE111 was constructed from pSBETc (Schenk et al., BioTechniques 19(2) (1995), 196-198).
pSBETc is a pACYC177-based expression vector that carries the argU gene, a kanamycin resistance gene and a T7 RNA polymerase promoter site for recombinant protein expression (Schenk et al., BioTechniques 19 (1995), 196 ff.). The helper plasmid pSE111 carries the lac repressor gene and the argU (dnaY) gene encoding a rare tRNA recognizing AGA and AGG arginine codons (Brinkmann et al., Gene 85 (1989), 109-114) and was constructed from pSBETc in two steps.
An Xmnl-EcoRV fragment, nucleotide position 2041-2521, was excised from pSBETc to remove the T7 promoter region.
A 1.2 kb EcoRl fragment containing the lacIQ gene was excised from plasmid pVH1 (Haring et al., Proc. Natl. Acad. Sci. USA 82 (1985), 6090-6094) and inserted into the unique EcoRl site of the plasmid resulting from step (i). Plasmids of 5.1 kb with laclQ inserts in both possible orientations were obtained; lin pSE111 transcription of the laclQ gene was clockwise in the published pSBETc map (Schenk et al., BioTechniques 19 (1995), 196 ff.). This plasmid was present in the E. coli strain SCS1 (Stratagene) used as the host strain for the cDNA expression library.
Using a picking/gridding robot, 80,640 clones were picked into 384-well microtiter plates and gridded at high density onto nylon and polyvinylidene difluoride (PVDF) filters. Nylon filters were processed for DNA hybridizations (DNA filters), whereas PVDF filters were transferred onto agar plates containing IPTG for induction of protein expression and processed for protein detection (protein filters).

### Example 2: Protein expression screening on high-density filters

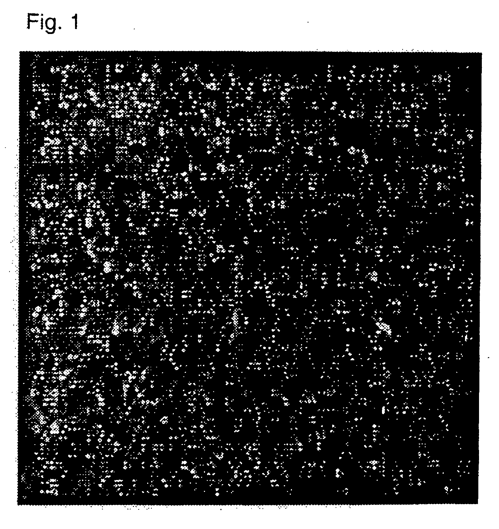

High-density protein filters of the hEx1 library were screened with the monoclonal RGS·His antibody recognizing the N-terminal sequence RGSH6 of recombinant fusion proteins overexpressed from the pQE-30NST vector. (Fig. 1). Approximately 20% of the clones were positive (signals of intensities 1, 2 or 3), classified one to three. These clones were considered putative protein expression clones (Fig. 1). The hEx1 cDNA library was prepared from human fetal brain tissues by oligo (dT) priming (Gubler et al., Gene 25 (1983), 263) using a Superscript Plasmid System kit (Life Technologies). cDNA was size-fractionated by gel filtration and individual fractions were ligated between the Sall and Notl sites of the expression vector pQE-30NST. E. coli SCS1 (Stratagene) carrying the helper plasmid pSE111 was used as the host strain. After transformation by electroporation, the library was plated onto square agar plates (Nunc Bio Assay Dish) and grown at 37°C overnight. Using an automated robotic system (Lehrach et al, Interdisciplinary Science Reviews 22 (1997), 37-44), colonies were picked into 384-well microtiter plates (Genetix) filled with 2×YT medium containing 100 µg/ml ampicillin, 15 µg/ml kanamycin, 2% glucose and freezing mix (0.4 mM MgS04, 1.5 mM Na3-citrate, 6.8 mM (NH4)2SO4, 3.6% glycerol, 13 mM KH2PO4, 27 mM K2HP04, [pH 7.0]). Bacteria were grown in the microtiter wells at 37°C overnight and replicated into new microtiter plates using 384-pin replicating tools (Genetix). All copies were stored frozen at 80°C.

### Example 3: Identification of genes and proteins on corresponding filter sets

GAPDH and HSP90a were chosen as example proteins, with open reading frames of 1,008 bp and 35,922 Dalton for GAPDH (Swiss-Prot P04406) and 2,199 bp and 84,542 Dalton for HSP90a (Swiss-Prot P07900).
A set of three high-density DNA filters (80,640 clones) of the hEx1 library was screened with gene-specific cDNA probes. High-density filters were prepared by robot spotting, as described (Maier et al., Drug Discovery Today 2 (1994), 315-324; Lehrach et al., Interdisciplinary Science Reviews 22 (1997), 37-44). Bacterial colonies were gridded onto Nylon membrane filters (Hybond N+, Amersham) for DNA analysis and on polyvinylidene difluoride (PVDF) membrane filters (Hybond-PVDF, Amersham) for protein analysis (filter format 222 mm × 222 mm). Clones were spotted at a density of 27,648 clones per filter in a duplicate pattern, surrounding ink guide dots. High-density filters were placed onto square 2×YT agar plates (Nunc Bio Assay Dish) containing 100 µg/ml ampicillin, 15 µg/ml kanamycin and 2% glucose.

Filters to be used for DNA analysis were grown overnight at 37°C and subsequently processed as previously described (Hoheisel et al., J. Mol. Biol. 220 (1991), 903-914). Filters for protein analysis were grown overnight at 30°C and subsequently then transferred onto agar plates supplemented with 1 mM IPTG to induce protein expression that was induced for 3 hours at 37°C. Expressed proteins were fixed on the filters by placing the filters onto blotting paper soaked in 0.5 M NaOH, 1.5 M NaCI for 10 minutes, twice for 5 minutes onto 1 M Tris-HCl, pH 7.5, 1.5 M NaCl for 5 minutes and finally onto 2×SSC for 15 minutes. Filters were air-dried and stored at room temperature.

DNA hybridizations using digoxigenin-labeled PCR probes and Attophos alkaline phosphatase substrate (JBL Scientific, San Luis Obispo) were performed as described (Maier et al., J. Biotechnol. 35 (1994), 191-203). Digoxigenin-labeled hybridization probes were prepared by PCR-amplification of a clone containing the complete open reading frame of human GAPDH and of the IMAGE clone number 343722 containing a C-terminal part of HSP90a (GenBank W69361).

With a human GAPDH probe (Fig. 2a), 206 (0.26%) clones were positive (Table 1) (Fig. 2a). A second hybridization confirmed 202 and detected 35 additional clones (raising the total to 237, Table 1). 56 (0.07%) clones were identified with a human HSP90a probe. On corresponding protein filters, 56 (27%) or 14 (25%) of GAPDH or HSP90a positive clones, respectively, were recognized by the RGS·His antibody.
Antibody screening on high-density filters was performed as follows: a rabbit anti-GAPDH serum was affinity purified as described (Gu et al., BioTechniques 17 (1994), 257-262). Anti-HSP90 (Transduction Laboratories, Lexington) is directed against amino acids 586 to 732 of HSP90a. Dry protein filters were soaked in ethanol, and bacterial debris was wiped off with paper towels in TBST-T (20 mM Tris-HCl, pH 7.5, 0.5 M NaCl, 0.05% Tween 20, 0.5% Triton X-100). The filters were blocked for 1 hour in blocking buffer (3% non-fat, dry milk powder in TBS, 150 mM NaCl, 10 mM Tris-HCl, pH 7.5) and incubated overnight with 50 ng/ml anti-HSP90 antibody or the anti-GAPDH antibody, diluted 1:5000. After two 10 minute washes in TBST-T and one in TBS, filters were incubated with alkaline phosphatase (AP)-conjugated secondary antibody for 1 hour. Following three 10 minute washes in TBST-T, one in TBS and one in AP buffer (1 mM MgCl2, 0.1 M Tris-HCl, pH 9.5), filters were incubated in 0.5 mM Attophos (JBL Scientific, San Luis Obispo) in AP buffer for 10 minutes. Filters were illuminated with long-wave UV light, and a high-resolution CCD detection system was used for image generation (Maier et al., Drug Discovery Today 2 (1997), 315-324). Positive clones were scored using custom-written image analysis software. With a polyclonal anti-GAPDH antibody (Fig. 2b), 39 clones were positive (Table 2). These were all detected by the RGS·His antibody but only 32 clones scored positive with the GAPDH-specific DNA probe. However, 5 of the 7 unaccounted clones were detected in the second DNA hybridization. Screening with a monoclonal anti-HSP90 antibody yielded 32 positive clones, 28 of which were detected by the HSP90a DNA probe, and 10 were positive with both the HSP90a DNA probe and the RGS·His antibody. In a second anti-HSP90 screening, 30 clones were confirmed, and 12 new clones were detected, which were all positive with the HSP90a DNA probe.

### Example 4: Sequence and Western blot analysis of detected clones

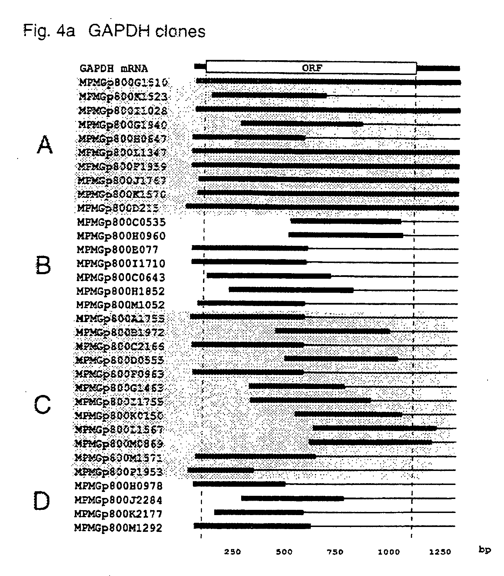

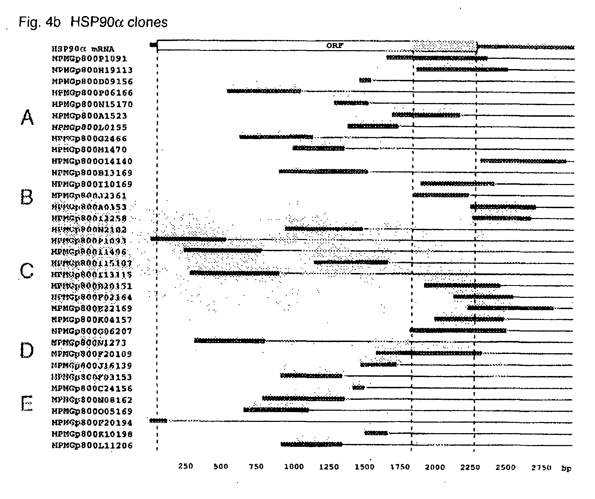

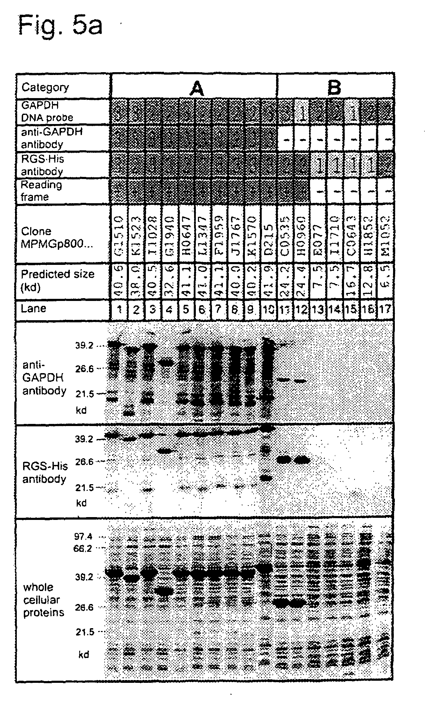

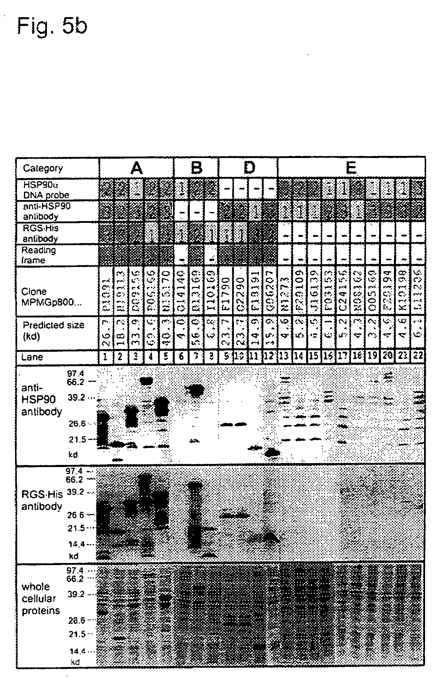

Fig. 3 summarizes the filter data obtained for GAPDH and HSP90a. Clones from categories A-E were analyzed by sequencing the 5'-ends of their cDNA inserts (Fig. 4) and by western blotting (Fig. 5). The following experimental protocols were carried out.

### (A) All-round positives

Ten GAPDH clones identified with the DNA probe, the anti-GAPDH and the RGS·His antibody were sequenced and found to contain GAPDH sequences in the correct reading frame. Nine clones expressed recombinant His6-tagged proteins spanning the full GAPDH sequence plus 5'-UTR and vector-amino acids encoded amino acids by the 5'-UTR of the mRNA and the vector.

All ten clones positive with the HSP90a DNA probe, the RGS·His and the anti-HSP90 antibody had HSP90a sequences in the correct reading frame. However, none of them accommodated the full coding region, and five clones were shown to express His6-tagged fusion proteins translated from differently sized C-terminal parts of the HSP90a sequence.

### (B) Specific antibody negatives

Sequences of seven GAPDH clones negative with the specific-GAPDH antibody on filters were shown to overlap the GAPDH GenBank sequence. Two of these clones had inserts in the correct reading frame and expressed GAPDH fragments (24 kD) that were stained by the anti-GAPDH antibody on western blots (Fig. 5a, B, lanes 11, 12). GAPDH inserts were in incorrect reading frames in the other five clones, suggesting expression of which supposedly expressed peptides in the range of 6.5- to 16.7 kD polypeptides (Fig. 5a, B, lanes 13-17). Signal intensities of these clones were generally low when probed with the RGS·His antibody on high-density filters. Three of four HSP90a clones had inserts in an incorrect reading frame, and expressed short peptides not reactive with the anti-HSP90 antibody (two clones shown in Fig. 5b, lanes 6, 8). The remaining clone carried an insert in the correct reading frame gave a band of the calculated size (56.0 kD) on western blots (Fig. 5b, lane 7) and was detected by the anti-HSP90 antibody in a second high-density filter screening.

### (C) DNA probe-only positives

Eleven out of twelve randomly selected GAPDH clones were shown to contained a GAPDH insert in an incorrect reading frame, supposedly expressing peptides in the range of 3.4 to 9.1 kD. Clone MPMGp800A1755 had an insert in the correct reading frame but carried a point mutation at position -8 in the 5'-UTR, leading to a stop codon and a calculated 4.7 kD peptide. DNA sequence analysis indicated that eleven out of twelve HSP90a clones contained inserts in an incorrect reading frame and possibly expressed peptides of 2.8- to 5.4 kD calculated molecular mass. Only clone MPMGp800I13115 had an insert in the correct reading frame, expressed a protein of 78.7 kD size and was positive in a second anti-HSP90 antibody screening.

No false positives were found for the GAPDH or the HSP90a DNA probe.

### (D) DNA probe negatives

Four GAPDH clones were shown to have correct inserts, representing false negatives of the DNA probe but were detected in a second DNA hybridization experiment. Two clones contained sequences of human polyubiquitin (GenBank D63791) and human HZF10 (PIR S47072).

All four HSP90a clones expressed polypeptides detected on western blots (Fig. 5b, D). Clone MPMGp800G06207 (lane 12) contained an HSP90a insert carrying a 46 bp deletion and was obviously a false negative of the HSP90a DNA probe. The remaining three clones accommodated inserts with sequence homology to murine uterine-specific proline-rich acidic protein (GenBank U28486; lanes 9, 10) or identity to an EST sequence of unknown function (lane 11).

### (E) DNA probe and specific antibody positives (RGS·His negatives)

Ten clones recognized by the HSP90a DNA probe and the anti-HSP90 antibody but not by the RGS·His antibody, were sequenced and found to contained HSP90a sequences inserted in an incorrect reading frame. His6-tagged polypeptides expressed from these clones would have calculated masses of 3.2- to 6.1 kD and were not found in western blots (Fig 5b, E). In contrast, matching patterns of bands were observed with the anti-HSP90 antibody.

Bacteria containing cDNA clones were grown by shaking in 2 ml 2×YT medium containing 100 µg/ml ampicillin, 15 µg/ml kanamycin and 2% glucose. At an O.D.600=0.4, IPTG was added to 1 mM final concentration, and the incubation was continued for 3 h at 37°C. Whole-cell protein extracts were subjected to 15% SDS-PAGE and stained with Coomassie blue, according to Laemmli (Laemmli, Nature 227 (1970), 680-685)

After SDS-PAGE, proteins were transferred onto PVDF membranes (Immobilon P, Millipore) in 20 mM Tris, 150 mM glycine, 0.1% SDS, 10% methanol, using a semi-dry electrotransfer apparatus (Hoefer Pharmacia Biotech, San Francisco). according to the manufacturer's recommendations.

cDNA inserts were amplified by PCR using primers pQE65 (TGA GCG GAT AAC AAT TTC ACA CAG) and pQE276 (GGC AAC CGA GCG TTC TGA AC) at an annealing temperature of 65°C. PCR products were sequenced using dye-terminator cycle sequencing with the pQE65 primer and ABI sequencers (Perkin Elmer) by the service department of our institute.

### Example 5: Vector constructs

pQE-30NST (GenBank accession number AF074376) has been described (Büssow et al., Nucleic Acids Res. 26 (1998), 5007-5008). pQE-BH6 was constructed using the polymerase chain reaction (PCR) for insertion of an oligonucleotide encoding the protein sequence LNDIFEAQKIEW between MRGS and His₆ of pQE-30 (Qiagen), thereby separating the two parts of the RGS- His₆ epitope.

### Example 6: Antibodies

Monoclonal antibodies of the following manufacturers were used at dilutions as indicated: mouse anti-RGS-His (QIAGEN, 1:2,000), mouse anti-rabbit GAPDH (Research Diagnostics Inc., clone 6C5, 1:5,000), mouse anti-HSP90 (Transduction Laboratories, clone 68, 1: 2,000), rat anti-alpha tubulin (Serotec Ltd., clone YL1/2, 1:2,000).
Secondary antibodies were F(ab')₂ rabbit anti-mouse lgG HRP (Sigma) and F(ab')₂ rabbit anti-rat IgG HRP (Serotec Ltd.), diluted 1:5,000, for the detection of mouse and rat monoclonals, respectively.

### Example 7: Large-scale protein expression and purification

Proteins were expressed in *E. coli* (strain SCS1) liquid cultures. 900 ml SB medium (12 g/l Bacto-tryptone, 24 g/l yeast extract, 17 mM KH₂PO₄, 72 mM K₂HPO₄, 0.4% (v/v) glycerol) containing 100 µg/ml ampicillin and 15 µg/ml kanamycin were inoculated with 10 ml of an overnight culture and shaken at 37°C until an OD₆₀₀ of 0.8 was reached. Isopropyl-b-D-thiogalactopyranosid (IPTG) was added to a final concentration of 1 mM. The culture was shaken for 3.5 h at 37°C and cooled to 4°C on ice. Cells were harvested by centrifugation at 2,100 g for 10 min, resuspended in 100 ml Phosphate Buffer (50 mM NaH₂PO₄, 0.3 M NaCl, pH 8.0) and centrifuged again. Cells were lysed in 3 ml per gram wet weight of Lysis Buffer (50 mM Tris, 300 mM NaCl, 0.1 mM EDTA, pH 8.0) containing 0.25 mg/ml lysozyme on ice for 30 min. DNA was sheared with an ultrasonic homogeniser (Sonifier 250, Branson Ultrasonics, Danbury, USA) for 3 x 1 min at 50% power on ice. The lysate was cleared by centrifugation at 10,000 g for 30 min. Ni-NTA agarose (Qiagen) was added and mixed by shaking at 4°C for 1 h. The mixture was poured into a column which was subsequently washed with ten bed volumes of Lysis Buffer containing 20 mM imidazole. Protein was eluted in Lysis Buffer containing 250 mM imidazole and was dialysed against TBS (10 mM Tris-HCl, 150 mM NaCl, pH 7.4) at 4°C overnight.

### Example 8: High-throughput small-scale protein expression

Proteins were expressed from selected clones of the arrayed human fetal brain cDNA expression library hEx1 (Büssow et al., Nucleic Acids Res. 26 (1998), 5007-5008). This library was directionally cloned in pQE-30NST for IPTG-inducible expression of His₆-tagged fusion proteins. 96-well microtitre plates with 2 ml cavities (StoreBlock, Zinsser) were filled with 100 µl SB medium, supplemented with 100 µg/ml ampicillin and 15 µl/ml kanamycin. Cultures were inoculated with *E. coli* SCS1 cells from 384-well library plates (Genetix, Christchurch, U.K.) that had been stored at -80°C. For inoculation, replicating devices carrying 96 steel pins (length 6 cm) were used. After overnight growth at 37°C with vigorous shaking, 900 µl of prewarmed medium were added to the cultures, and incubation was continued for 1 h. For induction of protein expression, IPTG was added to a final concentration of 1 mM. All following steps, including centrifugations, were also done in 96-well format. Cells were harvested by centrifugation at 1,900 g (3,400 rpm) for 10 min, washed by resuspension in Phosphate Buffer, centrifuged for 5 min and lysed by resuspension in 150 µl Buffer A (6 M Guanidinium-HCl, 0.1 M NaH₂PO₄, 0.01 M Tris-HCl, pH 8.0). Bacterial debris was pelleted by centrifugation at 4,000 rpm for 15 min. Supernatants were filtered through a 96-well filter plate containing a non-protein binding 0.65 µm pore size PVDF membrane (Durapore MADV N 65, Millipore) on a vacuum filtration manifold (Multiscreen, Millipore).

### Example 9: Automated filter spotting

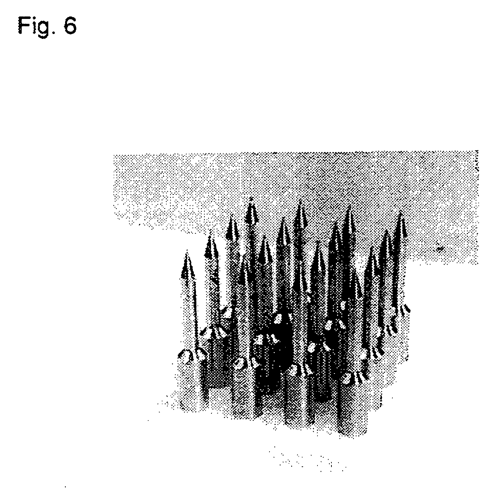

Pre-cut (25 x 75 mm) polyvinylidene difluoride (PVDF) filter strips (Immobilon P, Millipore) were soaked with 96 % ethanol and rinsed in distilled water for 1 min. Five wet filter strips were fixed with tape onto a 230 x 230 mm plastic tray. The spotting was done by a motor-carried transfer stamp (Fig. 6) which can be positioned at a resolution of 5 µm in x-y-z directions (Linear Drives, Basildon, UK). This allows densities of approximately 300 samples/cm², spotted in a duplicate pattern. The transfer stamp accommodates 4 x 4 = 16 individually mounted, spring-loaded pins at 4.5 mm spacing. Since the spacing is compatible to the spacing of 384-well plates, this tool enables high-density spotting out of 384-well microtitre plates. The size of the blunt-end tip of the stainless steel pins is 250 µm. Prior to each transfer, the spotting gadget was washed in a 30 % ethanol bath and subsequently dried with a fan to prevent cross contamination. For the experiments shown here, 4 x 4 patterns were spotted with each pin. Each pattern contains four ink guide spots surrounded by six samples spotted in duplicate (12 sample spots in total, Fig. 7A). Each spot was loaded five times with the same protein sample (5 nl each). Having adjusted the spotting height in advance, the spotting of 96 samples took approximately 20 min for the generation of five identical protein microarrays.

### Example 10:Antibody detection and image analysis

After spotting, filters were soaked in ethanol for 1 min, rinsed in distilled water, washed in TBST (TBS, 0.1 % Tween 20) for 1 min, blocked in 2% bovine serum albumin (BSA)/TBST for 60 min and incubated with monoclonal antibodies in 2% BSA/TBST for 1 h at room temperature, followed by two 10 min TBST washes and 1 h incubation with secondary antibodies in 2% BSA/TBST. Subsequently, filters were washed in 20 ml TBST overnight, incubated in 2 ml CN/DAB solution (Pierce) for 1-10 min, and positive reactions were detected as black spots. Images were acquired with a cooled CCD Camera (Fuji LHS, Raytest, Germany). Pictures were taken through a Fujinon objective (f: 0.8, 50 mm) with an integration time of 20 ms. image analysis was done with the AIDA package (Raytest, Germany) for spot recognition and quantification. The resulting spot values were transferred to an Excel spreadsheet (Microsoft, USA) to display the diagrammes of Figs. 7, 8 and 9.

### Example 11:Fabrication of protein microarrays

Proteins were expressed in liquid bacterial cultures, and solutions were spotted onto PVDF filters, either as crude lysates or after purification by Ni-NTA immobilised metal affinity chromatography (IMAC) (Hochuli et al, J. Chromatography. 411 (1987), 177-184). PVDF filter membranes were used for their superior protein binding capacity and mechanical strength (compared to nitrocellulose) and satisfactory former performance (Büssow et al., Nucleic Acids Res. 26 (1998), 5007-5008). The new transfer stamp (Fig. 6) consists of pins with 250 µm tip size, which is nearly half the size of the 450 µm pins that have previously been used for the generation of *in situ* protein expression filters (Büssow et al., Nucleic Acids Res. 26 (1998), 5007-5008). Although Figs. 7, 8 and 9, as our first test results, show about the same spotting density as our *in situ* filters, the smaller pin tip diameter enables higher spotting densities. While an *in situ* filter of 222 x 222 mm accommodates 27,648 clones (5 x 5 duplicate spotting pattern with one guide spot), more than 100,000 samples could be placed onto the same area using the new transfer stamp. This allows a substantial reduction in total array size to a convenient microscopic slide format (25 x 75 mm holding 4,800 samples, corresponding to 2,400 duplicates). The miniaturised set-up allows a very economic use and high concentrations of reagents in incubating solutions as a much smaller buffer volume is needed to cover the filters. In contrast to *in situ* filters, the signals obtained on microarrays are sharp and well localised. As the next step towards the fabrication of protein chips, we envisage a further increase in density by using high-speed picolitre spotting (inkjetting) onto modified glass surfaces. Alternative approaches to protein microarrays have been reported using either photolithography of silane monolayers (Mooney et al., Proc. Natl. Acad. Sci. USA. 93 (1996), 12287-12291) or inkjetting onto polystyrene film (Ekins, Clin. Chem. 44 (1998), 2015-2030; Silzel et al., Clin. Chem. 44 (1998), 2036-2043). In contrast to our library spotting technology, those advances have been focused on the fabrication of miniaturised immunoassay formats by patterning of single proteins (e.g., BSA, avidin or anti-lgG monoclonal antibodies).

### Example 12: Sensitivity of specific protein detection

The sensitivity of specific protein detection on microarrays was assessed by spotting different concentrations of three purified proteins, human glyceraldehyde-3-phosphate dehydrogenase (GAPDH, Swiss-Prot P04406), a C-terminal fragment (40.3 kd) of human heat shock protein 90 alpha (HSP90alpha, Swiss-Prot P07900) and rat immunoglobulin heavy chain binding protein (BIP, Swiss-Prot P06761). Microarrays were subsequently incubated with a monoclonal anti-GAPDH antibody, rabbit anti-mouse IgG HRP and HRP substrate CN/DAB (Fig. 7A). The sensitivity of detection, as the lowest concentration that delivered clearly visible, specific spots above background (detection threshold), was calculated to be 10 fmol/µl, corresponding to 250 attomol or 10 pg of GAPDH in 5 x 5 nl spotted (Fig. 7B).

### Example 13:High-throughput screening for protein expression

Crude lysates of 92 clones of the arrayed human fetal brain cDNA library hEx1 (Büssow et al., Nucleic Acids Res. 26 (1998), 5007-5008), previously identified as protein expressors by the monoclonal antibody RGS-His (Qiagen) on *in situ* filters, were spotted in duplicate, alongside with 4 control samples and ink guide spots. Microarrays were screened for expression of RGS-His₆-tagged fusion proteins using the same antibody (Fig. 8A, insert). When relative intensities of duplicates (see Fig. 7A) are plotted against each other, the resulting diagonal indicates a good reproducibility of the detection method (Fig. 8A). Therefore, means of duplicates were plotted for all 96 samples, and an arbitrary specificity threshold for identification of positives was set to 7,500 relative intensity (Fig. 8B). Under these conditions, a negative control (H1, vector pQE-30NST without insert) was clearly negative (1,500 relative intensity), as was an HSP90alpha clone, featuring a divided RGS-His₆ epitope (H2, vector pQE-BH6; 0 relative intensity). The lysate of an RGS-His₆-tagged GAPDH clone (H3, vector pQE-30NST) was used as a positive control and delivered a signal of 21,000 relative intensity. The clearly positive result (15,000 relative intensity) obtained with a rat BIP clone (H4, vector pQE-BH6) is surprising because this clone also features a divided RGS-His₆ epitope. The reactivity might be explained by partial re-constitution of the RGS-His₆ epitope due to conformational characteristics of BIP.
The cDNA inserts of 54 of the 92 putative hEx1 expression clones show homology to Genbank entries of human genes (Büssow, Thesis, Department of Chemistry, Free University Berlin (1998)). These inserts were checked for their reading frames (RF) in relation to the vector-encoded RGS-His₆ tag sequence. 34 inserts (63%) were found to be cloned in the correct reading frame (RF+), while 20 (37%) were in an incorrect reading frame (RF-), hence those clones could not be expected to express the predicted protein. However, all 92 clones were originally selected as protein expressors on *in situ* filters due to clearly positive signals with the monoclonal antibody RGS-His [intensity levels 2 and 3, (Büssow, Thesis, Department of Chemistry, Free University Berlin (1998))]. On microarrays, the number of incorrect reading frame clones identified as protein expressors was decreased by 70%, as only 6 RF(-) clones were still confirmed as positives (Fig. 8B). This indicates that the new microarray technology is a major advancement over *in situ* filters for its superior ability to exclude incorrect reading frame clones. On the other hand, only one RF(+) clone was clearly below the specificity threshold and would have been missed in this screen, probably due to an insufficient amount of protein expressed in the microtitre well. This stresses again the nature of our approach that is exclusively based on "positives" to be confirmed by sequencing and/or protein characterisation (Büssow et al., Nucleic Acids Res. 26 (1998), 5007-5008).
In summary, the high-throughput protein expression screening on microarrays resulted in a false negative rate of under 2% (1 undetected RF(+) clone per 54 clones total). The rate of false positive clones, expressing proteins in incorrect reading frames, was down to 11%, compared to 37% on *in situ* filters (Büssow, Thesis, Department of Chemistry, Free University Berlin (1998). That makes protein microarrays an economical tool for very sensitive protein expression screening.

### Example 14:Antibody specificity screening

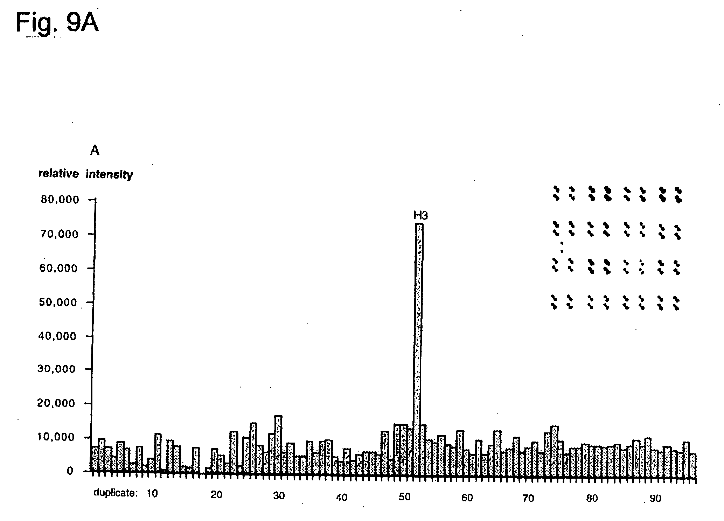

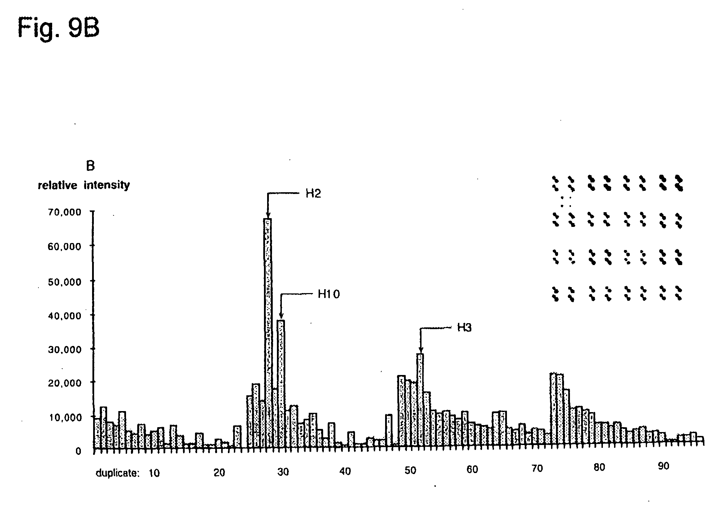

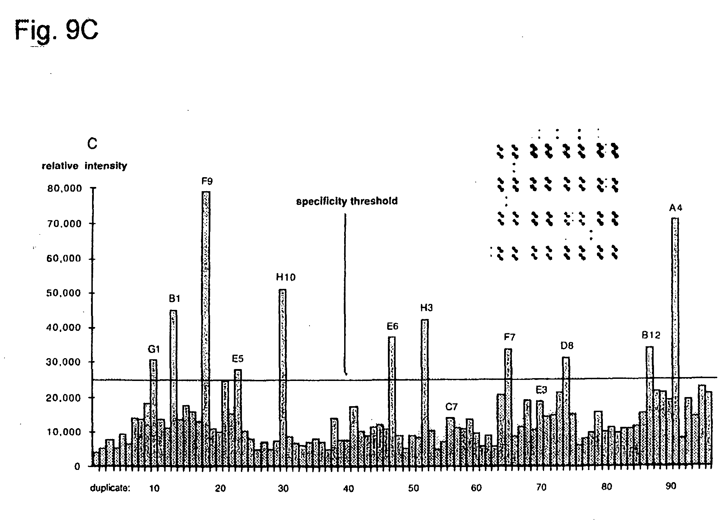

Protein microarrays featuring the same test set of 92 hEx1 expression clones and 4 controls (see above) were screened for the human proteins GAPDH, HSP90alpha and alpha tubulin using monoclonal antibodies. While the anti-GAPDH antibody detected its target antigen exclusively (H3, Fig. 9A), anti-HSP90alpha preferentially recognised its target antigen (H2, Fig. 9B) but showed some cross-reactivity with at least two other clones (H10, 60S ribosomal protein L18A and H3, GAPDH). Antibody cross-reactivity was even more pronounced in the anti-alpha tubuiin screen (Fig. 9C). While the two RF(+) alpha tubulin clones in the test set (F9 and A4) were specifically recognised and the only RF(-) clone (C7) was left undetected, nine other clones showed anti-alpha tubulin reactivity above the arbitrary specificity threshold. Two of these clones (B1 and B12) represent unknown genes, and G1 is an RF(-) beta tubulin clone. H3 is the GAPDH positive control clone of Fig. 8 (see above), which to some extent seems to cross-react unspecifically (Figs. 9B and 9C), possibly due to an exceptionally high level of protein expression. Surprisingly, all other (five) clones above threshold express ribosomal proteins in a correct reading frame (E5, RPL3; H10, RPL18A; E6 and D8, RPS2; F7, RPS3A). Only one additional ribosomal protein in the test set (E3, RPS25) did not show an anti-alpha tubulin reactivity. The epitope recognised by the anti-alpha tubulin antibody (YL1/2, (Kilmartin et al., J. Cell Biol. 93 (1982), 576-582)) was identified as the linear sequence spanning the carboxy-terminal residues of tyrosinated alpha tubulin (Wehland et al., EMBO J. 3 (1984), 1295-1300). According to those authors, the minimal sequence requirements, as defined by dipeptide studies, are a negatively charged side chain in the penultimate position followed by an aromatic residue which must carry the free carboxylate group. As none of the cross-reacting ribosomal proteins on our microarrays fulfill these requirements, other (e.g., structural) epitopes might mimic the antigenic specificity.

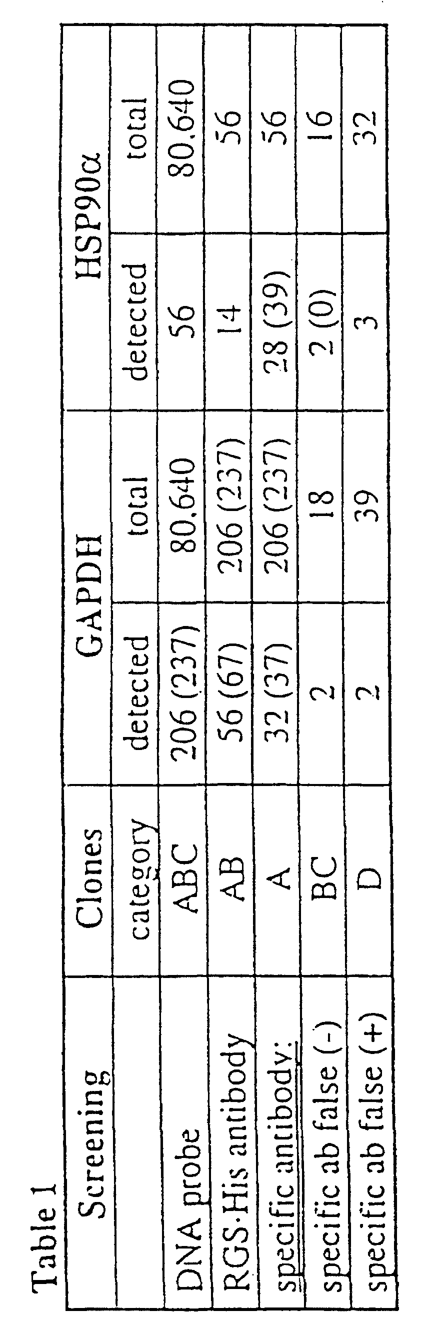

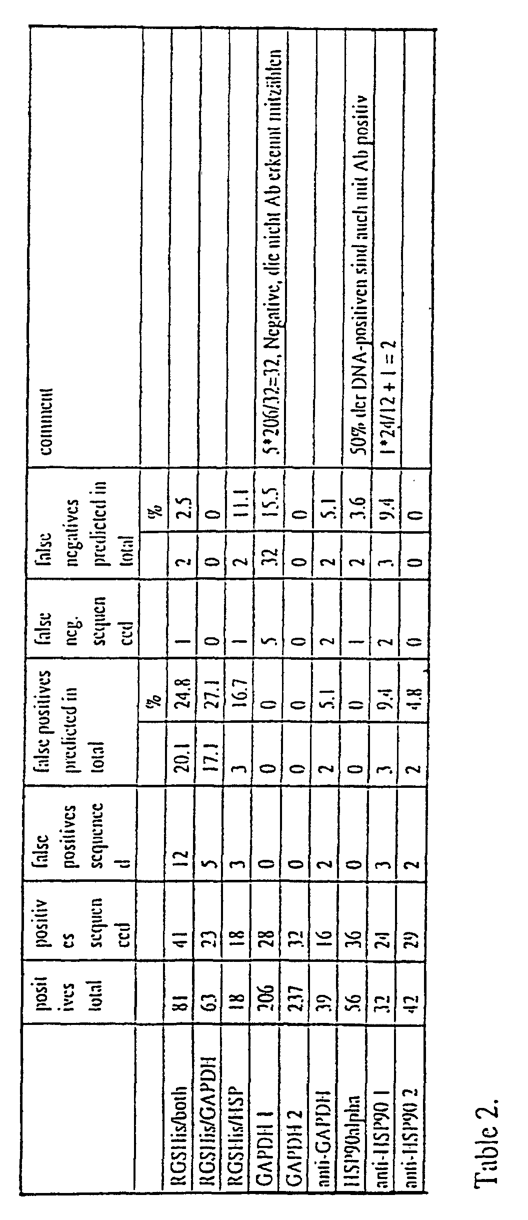

## Claims

1. A method for the identification and/or characterization of clones of an expression library, said clones conferring a desired biological property comprising the following steps
(a) analyzing for the expression of at least one (poly)peptide expressed as a fusion protein with an expression product of a recombinant insert of a clone of said expression library, the clones of said expression library being arranged in arrayed form; and
(b) contacting a ligand specifically interacting with a (poly)peptide expressed by the insert of a clone conferring said desired biological property with said library or a first replica of said library of clones in arrayed form and analyzing said library of clones for the occurrence of an interaction; and/or
(c) carrying out a hybridization or an oligonucleotide fingerprint with a nucleic acid probe specific for the insert of a clone conferring said desired biological property with said library or said first replica or a second replica of said library of clones arranged in arrayed form and analyzing said library of clones for the occurrence of a hybridization; and
(d) identifying and/or characterizing clones wherein an expression of the at least one (poly)peptide in step (a) and/or an interaction in step (b) and/or a specific hybridization or an oligonucleotide fingerprint in step (c) can be detected.

2. The method of claim 1, wherein said (poly)peptide expressed as a part of a fusion protein with said expression product of said recombinant insert is an antibody or a fragment or derivative thereof, wherein said fragment is a F(ab')₂, Fab, Fv or scFv fragment or an antibody modified such as by deletion of certain parts thereof but essentially maintaining its capacity to function as a ligand, a tag, an enzyme, a phage protein or a fusion protein.

3. The method of claim 1 or 2, wherein said analysis for the expression of a (poly)peptide in step (a) is effected by contacting a ligand different from the ligand of step (b) that specifically interacts with said (poly)peptide and analyzing said library of clones for a specific interaction to occur.

4. The method of any one of claims 1 to 3, wherein said analysis for the expression of a (poly)peptide in step (a) is effected by visual means, preferably mass spectrometry.

5. The method of any one of claims 1 to 4, wherein said desired biological property is specificity for a cell, a tissue, or the developmental stage of a cell or a tissue, a microorganism, preferably a bacterium, a plant or an organism.

6. The method of claim 5, wherein said cell or tissue is a normal cell or tissue, a diseased cell or tissue, or a pretreated cell or tissue.

7. The method of any one of claims 1 to 6, wherein said clones are bacterial transformants, recombinant phage, transformed mammalian, insect, fungal, yeast or plant cells.

8. The method of any one of claims 1 to 7, wherein said arrayed form has substantially the same format in steps (a) to (c).

9. The method of any one of claims 1 to 8, wherein said arrayed form is a grid form.

10. The method of claim 9, wherein said grid has the dimensions of a microtiter plate, a silica wafer, a chip, a mass spectrometry target or a matrix.

11. The method of any one of claims 1 to 10, wherein said clones are affixed to a solid support.

12. The method of claim 11, wherein said solid support is a filter, a membrane, a magnetic bead, a silica wafer, glass, metal, a chip, a mass spectrometry target or a matrix.

13. The method of any one of claims 1 to 12, wherein at least one of said ligands is a (poly)peptide, a phage, blood, serum, a toxin, an inhibitor, a drug or a drug candidate, a non-proteinaceous or partially proteinaceous receptor, a catalytic polymer, an enzyme, a nucleic acid, a PNA, a virus, a cell, an inorganic compound, a conjugate, a dye, a tissue or a conjugate of said ligand.

14. The method of claim 13, wherein said (poly)peptide is an antibody or a F(ab')₂, Fab, Fv or scFv fragment thereof, a hormone or an enzyme, or an antibody, hormone or enzyme modified such as by deletion of certain parts thereof but essentially maintaining their capacity to function as a ligand.

15. The method of any one of claims 1 to 14, wherein said interaction in step (b) is a specific interaction.

16. The method of any one of claims 1 to 14, wherein said interaction in step (b) is an unspecific interaction.

17. The method of any one of claims 1 to 16, wherein said hybridization in step (c) occurs under stringent conditions.

18. The method of any one of claims 1 to 16, wherein said hybridization in step (c) occurs under non-stringent conditions.

19. The method of any one of claims 2 to 18, wherein said tag is c-myc, His-tag, FLAG, alkaline phosphatase, V5 tag, T7 tag, or Strep-tag, a fusion protein, preferably GST, cellulose binding domain, green fluorescent protein, maltose binding protein or lacZ.

20. The method of any one of claims 1 to 19, wherein said library of clones comprises a cDNA library.

21. The method of any one of claims 1 to 20, wherein said arrayed form of said library and/or said replicas is/are generated by an automated device.

22. The method of claim 21, wherein said automated device is a picking robot and/or spotting robot and/or gridding robot

23. The method of any one of claims 1 to 22 further comprising sequencing the nucleic acid insert of said desired clone.

24. The method of any one of claims 1 to 22 further comprising identifying and/or characterizing the (poly)peptide encoded by the insert of the desired clone.

25. Kit comprising at least two replicas of expression libraries, wherein at least one (poly)peptide is expressed as a fusion protein with a recombinant insert of a clone of said expression library and clones of said expression library confer a desired biological property, affixed to a solid support, wherein the clones of such expression libraries are arrayed in grid form.

26. Kit according to claim 25, wherein one of said replicas comprises (poly)peptides expressed by the inserts of said clones and a further replica comprises a genomic or a cDNA library.

27. An array comprising an expression library of clones, wherein at least one (poly)peptide .is expressed as a fusion protein with a recombinant insert of a clone of said expression library and clones of said expression library confer a desired biological property, wherein the arrayed form is a grid form.

## Patentansprüche

1. Verfahren zur Identifizierung und/oder Charakterisierung von Clonen aus einer Expressionsbibliothek, wobei die Clone eine gewünschte biologische Eigenschaft verleihen, umfassend die folgenden Schritte:
(a) Analysieren auf die Expression von mindestens einem (Poly)Peptid, exprimiert als Fusionsprotein mit einem Expressionsprodukt eines rekombinanten Inserts eines Clons der Expressionsbibliothek, wobei die Clone der Expressionsbibliothek in einer Anordnung arrangiert sind; und
(b) Inkontaktbringen eines Liganden, der mit einem Polypeptid spezifisch interagiert, das durch das Insert eines Clons exprimiert wird, der die gewünschte biologische Eigenschaft verleiht, mit der Bibliothek oder einer ersten Kopie der Bibliothek von Clonen in einer Anordnung und Analysieren der Clone der Bibliothek auf das Vorliegen einer Interaktion; und/oder
(c) Durchführen einer Hybridisierung oder eines Oligonucleotid-Fingerprints mit einer für das Insert des Clons, der die gewünschte biologische Eigenschaft verleiht, spezifischen Nucleinsäure-Sonde mit der Bibliothek oder einer ersten Kopie oder einer zweiten Kopie der Bibliothek von Clonen, angeordnet in einer Anordnung und Analysieren der Bibliothek von Clonen auf das Vorliegen einer Hybridisierung; und
(d) Identifizieren und/oder Charakterisieren von Clonen, wobei eine Expression des mindestens einen (Poly)Peptids in Schritt (a) und/oder eine Interaktion in Schritt (b) und/oder eine spezifische Hybridisierung oder ein Oligonucleotid-Fingerprint in Schritt (c) nachgewiesen werden kann.

2. Verfahren nach Anspruch 1, wobei das (Poly)Peptid, welches als Teil eines Fusionsproteins mit dem Expressionsprodukt des rekombinanten Inserts exprimiert wird, ein Antikörper oder ein Fragment oder ein Derivat eines solchen, wobei das Fragment ein F(ab')₂, Fab, Fv oder scFv-Fragment ist oder ein Antikörper modifiziert durch Deletion bestimmter Teile davon im Wesentlichen unter Aufrechterhaltung seiner Funktionsfähigkeit als Ligand ist, ein Tag, ein Enzym, ein Phagen-Protein oder ein Fusionsprotein ist.

3. Verfahren nach einem der Ansprüche 1 oder 2, wobei die Analyse auf die Expression eines (Poly)Peptids in Schritt (a) durchgeführt wird durch Inkontaktbringen eines Liganden, der unterschiedlich von dem Liganden aus Schritt (b) ist, der spezifisch mit dem (Poly)Peptid interagiert, und Analysieren der Bibliothek von Clonen, ob eine spezifische Interaktion vorkommt.

4. Verfahren nach einem der Ansprüche 1 bis 3, wobei die Analyse auf die Expression eines (Poly)Peptids in Schritt (a) mit optischen Mitteln, vorzugsweise durch Massenspektrometrie, durchgeführt wird.

5. Verfahren nach einem der Ansprüche 1 bis 4, wobei die gewünschte biologische Eigenschaft Spezifität für eine Zelle, ein Gewebe, ein Entwicklungsstadium einer Zelle oder eines Gewebes, einen Mikroorganismus, vorzugsweise ein Bakterium, eine Pflanze oder ein Organismus ist.

6. Verfahren nach Anspruch 5, wobei die Zelle oder das Gewebe eine normale Zelle oder normales Gewebe, eine erkrankte Zelle oder erkranktes Gewebe oder eine vorbehandelte Zelle oder vorbehandeltes Gewebe ist.

7. Verfahren nach einem der Ansprüche 1 bis 6, wobei die Clone transformierte Bakterien, rekombinante Phagen, oder transformierte Zellen von Säugern, Insekten, Pilzen, Hefen oder Pflanzen sind.

8. Verfahren nach einem der Ansprüche 1 bis 7, wobei die Anordnung im Wesentlichen jener in den Schritten (a) bis (c) entspricht.

9. Verfahren nach einem der Ansprüche 1 bis 8, wobei die Anordnung einem Gitter entspricht.

10. Verfahren nach Anspruch 9, wobei das Gitter die Größenordnung einer Mikrotiterplatte, eines Silika-Wafers, eines Chips, eines massenspektrometrischen Targets oder einer Matrix besitzt.

11. Verfahren nach einem der Ansprüche 1 bis 10, wobei die Clone auf einem festen Träger fixiert sind.

12. Verfahren nach Anspruch 11, wobei der feste Träger ein Filter, eine Membran, ein magnetisches Kügelchen, ein Silika-Wafer, Glas, Metall, ein Chip, ein massenspektrometrisches Target oder eine Matrix ist.

13. Verfahren nach einem der Ansprüche 1 bis 12, wobei mindestens einer der Liganden ein (Poly)Peptid, ein Phage oder ein Fragement davon, Blut, Serum, ein Toxin, ein Inhibitor, ein Wirkstoff oder ein Wirkstoff-Kandidat, ein nicht proteinartiger oder zum Teil proteinartiger Rezeptor, ein katalytisches Polymer, ein Enzym, eine Nucleinsäure, eine PNA, ein Virus oder ein Teil davon, eine Zelle oder ein Teil davon, eine anorganische Verbindung, ein Konjugat, ein Farbstoff, ein Gewebe oder ein Konjugat des Liganden ist.

14. Verfahren nach Anspruch 13, wobei das (Poly)Peptid ein Antikörper oder ein Fragment oder Derivat davon, ein Hormon oder ein Fragment davon oder ein Enzym oder ein Fragment oder ein Derivat davon ist.

15. Verfahren nach einem der Ansprüche 1 bis 14, wobei die Interaktion in Schritt (b) eine spezifische Interaktion ist.

16. Verfahren nach einem der Ansprüche 1 bis 14, wobei die Interaktion in Schritt (b) eine unspezifische Interaktion ist.

17. Verfahren nach einem der Ansprüche 1 bis 16, wobei die Hybridisierung in Schritt (c) unter stringenten Bedingungen zustande kommt.

18. Verfahren nach einem der Ansprüche 1 bis 16, wobei die Hybridisierung in Schritt (c) unter nicht-stringenten Bedingungen zustande kommt.

19. Verfahren nach einem der Ansprüche 2 bis 18, wobei das Tag c-myc, His-Tag, FLAG, alkalische Phosphatase, EpiTAG™, V5-Tag, T7-Tag, Xpress™-Tag oder Strep-Tag, ein Fusionsprotein, vorzugsweise GST, eine Cellulose-bindende Domäne, grünfluoreszierendes Protein, Maltose bindendes Protein oder lacZ ist.

20. Verfahren nach einem der Ansprüche 1 bis 19, wobei die Bibliothek von Clonen eine cDNA-Bibliothek umfasst.

21. Verfahren nach einem der Ansprüche 1 bis 20, wobei die Anordnung der Bibliothek und/oder die Kopien mit einer automatischen Vorrichtung hergestellt wird/werden.

22. Verfahren nach Anspruch 21, wobei die automatische Vorrichtung ein Sortierautomat und/oder ein Tropfenautomat und/oder ein Rasterautomat ist.

23. Verfahren nach einem der Ansprüche 1 bis 22, darüber hinaus umfassend die Sequenzierung des Nucleinsäureinserts des gewünschten Clons.

24. Verfahren nach einem der Ansprüche 1 bis 22, darüber hinaus umfassend die Identifizierung und/oder Charakterisierung des (Poly)Peptids, das von dem Insert des gewünschten Clons codiert wird.

25. Kit umfassend mindestens zwei Kopien von Expressionsbibliotheken, wobei mindestens ein (Poly)petid ist exprimiert als ein Fusionsprotein mit einem rekombinanten Insert eines Clons einer solchen Expressionsbibliothek und Klone dieser Expressionsbibliothek eine gewünschte Eigenschaft verleihen, fixiert an einen festen Träger, wobei die Klone dieser Expressionsbibliothek in Gitterform angeordnet sind.

26. Kit nach Anspruch 25, wobei eine der Kopien (Poly)Peptide umfasst, die durch Inserts dieser Clone exprimiert werden, und eine weitere Kopie eine genomische oder cDNA-Bibliothek umfasst.

27. Eine Anordnung umfassend eine Expressionsbibliothek aus Clone, wobei mindestens ein (Poly)petid ist exprimiert als ein Fusionsprotein mit einem rekombinanten Insert eines Clons einer solchen Expressionsbibliothek und Klone dieser Expressionsbibliothek eine gewünschte Eigenschaft verleihen, wobei die angeordnete Form eine Gitterform ist.

## Revendications

1. Procédé d'identification et/ou de caractérisation de clones d'une bibliothèque d'expression, lesdits clones conférant une propriété biologique désirée comprenant les étapes de :
a) analyse de l'expression d'au moins un (poly)peptide exprimé comme protéine de fusion avec un produit d'expression d'un insérat recombinant d'un clone de ladite bibliothèque d'expression, les clones de ladite bibliothèque d'expression étant agencés sous forme alignée ; et
b) mise en contact d'un ligand par interaction spécifique avec un (poly)peptide exprimé par l'insérat d'un clone conférant ladite propriété biologique désirée avec ladite bibliothèque ou une première réplique de ladite bibliothèque de clones sous forme alignée et analyse de ladite bibliothèque de clones pour déceler l'apparition d'une interaction ; et/ou
c) réalisation d'une hybridation ou d'une empreinte oligonucléotidique avec une sonde d'acide nucléique spécifique à l'insérat d'un clone conférant ladite propriété biologique désirée avec ladite bibliothèque ou ladite première réplique ou une seconde réplique de ladite bibliothèque de clones agencés sous forme alignée et analyse de ladite bibliothèque de clones pour déceler l'apparition d'une hybridation ; et
d) identification et/ou caractérisation de clones dans laquelle (lesquelles) une expression du au moins un (poly)peptide dans l'étape (a) et/ou une interaction dans l'étape (b) et/ou une hybridation ou une empreinte oligonucléotidique spécifique dans l'étape (c) peut être détectée.

2. Procédé de la revendication 1, dans lequel ledit poly)peptide exprimé comme partie d'une protéine de fusion avec ledit produit d'expression dudit insérat recombinant est un anticorps ou un fragment ou dérivé de celui-ci, dans lequel ledit fragment est un fragment F(ab')₂, Fab, Fv ou scFv ou un anticorps modifié tel que par délétion de certaines parties de celui-ci mais en conservant essentiellement sa capacité à fonctionner comme ligand, étiquette, enzyme, protéine phagique ou protéine de fusion.

3. Procédé de la revendication 1 ou 2, dans lequel ladite analyse pour l'expression d'un (poly)peptide dans l'étape (a) est effectuée par mise en contact d'un ligand différent du ligand de l'étape (b) qui interagit de manière spécifique avec ledit (poly)peptide et par analyse de ladite bibliothèque de clones pour déceler l'apparition d'une interaction spécifique.

4. Procédé de l'une quelconque des revendications 1 à 3, dans lequel ladite analyse pour l'expression d'un (poly)peptide dans l'étape (a) est effectuée par des moyens visuels, spectrométrie de masse, de préférence.

5. Procédé de l'une quelconque des revendications 1 à 4, dans lequel ladite propriété biologique désirée est spécifique à une cellule, un tissu ou à l'étape de développement d'une cellule ou d'un tissu, un microorganisme, bactérie, de préférence, une plante ou un organisme.

6. Procédé de la revendication 5, dans lequel ladite cellule ou ledit tissu est une cellule ou un tissu normal(e), une cellule ou un tissu malade ou une cellule ou un tissu prétraité(e).

7. Procédé de l'une quelconque des revendications 1 à 6, dans lequel lesdits clones sont des transformants bactériens, un phage recombinant, un mammifère transformé, un insecte, un champignon, de la levure ou des cellules de plantes.

8. Procédé de l'une quelconque des revendications 1 à 7, dans lequel ladite forme alignée a sensiblement le même format que dans les étapes (a) à (c).

9. Procédé de l'une quelconque des revendications 1 à 8, dans lequel ladite forme alignée est une forme en grille.

10. Procédé de la revendication 9, dans lequel ladite grille a les dimensions d'une plaque de microtitrage, d'une tranche de silice, d'une puce, d'une cible ou d'une matrice de spectrométrie de masse.

11. Procédé de l'une quelconque des revendications 1 à 10, dans lequel lesdits clones sont fixés à un support solide.

12. Procédé de la revendication 11, dans lequel ledit support solide est un filtre, une membrane, une bille magnétique, une tranche de silice, du verre, du métal, une puce, une cible ou une matrice de spectrométrie de masse.

13. Procédé de l'une quelconque des revendications 1 à 12, dans lequel au moins un desdits ligands est un (poly)peptide, un phage, du sang, du sérum, une toxine, un inhibiteur, un médicament ou un candidat médicament, un récepteur partiellement protéique ou non protéique, un polymère catalytique, une enzyme, un acide nucléique, une PNA (agglutinine d'arachide), un virus, une cellule, un composé inorganique, un conjugué, un colorant, un tissu ou un conjugué dudit ligand.

14. Procédé de la revendication 13, dans lequel ledit (poly)peptide est un anticorps ou un fragment F(ab')₂, Fab, Fv ou scFv de celui-ci, une hormone ou une enzyme, ou un anticorps, hormone ou enzyme modifié(e) tel(le) que par délétion de certaines parties de celui-ci (de celle-ci) mais en conservant essentiellement sa capacité à fonctionner comme ligand.

15. Procédé de l'une quelconque des revendications 1 à 14, dans lequel ladite interaction de l'étape (b) est une interaction spécifique.

16. Procédé de l'une quelconque des revendications 1 à 14, dans lequel ladite interaction de l'étape (b) est une interaction non spécifique.

17. Procédé de l'une quelconque des revendications 1 à 16, dans lequel ladite hybridation de l'étape (c) se produit dans des conditions stringentes.

18. Procédé de l'une quelconque des revendications 1 à 16, dans lequel ladite hybridation de l'étape (c) se produit dans des conditions non stringentes.

19. Procédé de l'une quelconque des revendications 2 à 18, dans lequel ladite étiquette est c-myc, une étiquette His, un DRAPEAU, une phosphatase alcaline, une étiquette V5, une étiquette T7 ou une étiquette Strep, une protéine de fusion, de préférence, GST, un domaine de liaison à la cellulose, une protéine fluorescente verte, une protéine de liaison au maltose ou lacZ.

20. Procédé de l'une quelconque des revendications 1 à 19, dans lequel ladite bibliothèque de clones comprend une bibliothèque d'ADNc.

21. Procédé de l'une quelconque des revendications 1 à 20, dans lequel ladite forme alignée de ladite bibliothèque et/ou desdites répliques est/sont générée(s) par un dispositif automatique.

22. Procédé de la revendication 21, dans lequel ledit dispositif automatique est un robot de prélèvement et/ou un robot de repérage et/ou de robot de quadrillage.

23. Procédé de l'une quelconque des revendications 1 à 22 comprenant, en outre, le séquençage de l'insérat d'acide nucléique dudit clone désiré.

24. Procédé de l'une quelconque des revendications 1 à 22 comprenant, en outre, l'identification et/ou la caractérisation du poly(peptide) codé par l'insérat du clone désiré.

25. Kit comprenant au moins deux répliques de bibliothèques d'expression, dans lequel au moins un (poly)peptide est exprimé comme protéine de fusion avec un insérat recombinant d'un clone de ladite bibliothèque d'expression et dans lequel des clones de ladite bibliothèque d'expression confèrent une propriété biologique désirée, fixés à un support solide, dans lequel les clones de telles bibliothèques d'expression sont disposés sous forme de grille.

26. Kit selon la revendication 25, dans lequel une desdites répliques comprend des (poly)peptides exprimés par les insérats desdits clones et dans lequel une autre réplique comprend une bibliothèque d'ADNc ou une banque génomique.

27. Agencement comprenant une bibliothèque d'expression de clones, dans lequel au moins un poly(peptide) est exprimé comme protéine de fusion avec un insérat recombinant d'un clone de ladite bibliothèque d'expression et dans lequel des clones de ladite bibliothèque d'expression confèrent une propriété biologique désirée, dans lequel la forme alignée est une forme de grille.
